# EUROPÄISCHE PATENTSCHRIFT

(11) **EP 1 311 658 B1**
(45) Veröffentlichungstag und Bekanntmachung des Hinweises auf die Patenterteilung: **15.10.2008**
(21) Anmeldenummer: 01960708.4
(22) Anmeldetag: 24.08.2001
(51) Int. Cl.: C12N 5/08, A01N 1/02

(54) **VERFAHREN ZUR HERSTELLUNG GEBRAUCHSFERTIGER, ANTIGENBELADENER ODER -UNBELADENER, KRYOKONSERVIERTER, REIFER DENDRITISCHER ZELLEN**
METHOD FOR PRODUCING READY TO USE, ANTIGEN LOADED OR UNLOADED, CRYOCONSERVED MATURE DENDRITIC CELLS
PROCEDE DE FABRICATION DE CELLULES DENDRITIQUES MATURES, CRYOCONSERVEES, CHARGEES D'ANTIGENES OU NON, PRETES A L'EMPLOI

(30) Priorität: 24.08.2000 DE 10041515
(43) Veröffentlichungstag der Anmeldung: 21.05.2003
(73) Patentinhaber: Argos Therapeutics, Inc., Durham, NC 27704 (US)
(72) Erfinder: SCHULER, Gerold, 91080 Spardorf (DE); SCHULER-THURNER, Beatrice, 91080 Spardorf (DE)
(74) Vertreter: Helbing, Jörg
(86) Internationale Anmeldenummer: PCT/EP2001/009790
(87) Internationale Veröffentlichungsnummer: WO 2002/016560

(56) Entgegenhaltungen:
- WO-A-99/46984
- DATABASE BIOSIS [Online] BIOSCIENCES INFORMATION SERVICE, PHILADELPHIA, PA, US; 1997 MAKINO M ET AL: "A cryopreservation method of human peripheral blood mononuclear cells for efficient production of dendritic cells." Database accession no. PREV199799644877 XP002186870 & SCANDINAVIAN JOURNAL OF IMMUNOLOGY, Bd. 45, Nr. 6, 1997, Seiten 618-622, ISSN: 0300-9475
- LEWALLE P ET AL: "Freezing of dendritic cells, generated from cryopreserved leukaphereses, does not influence their ability to induce antigen-specific immune responses or functionally react to maturation stimuli" JOURNAL OF IMMUNOLOGICAL METHODS, ELSEVIER SCIENCE PUBLISHERS B.V.,AMSTERDAM, NL, Bd. 240, Nr. 1-2, Juni 2000 (2000-06), Seiten 69-78, XP004201567 ISSN: 0022-1759 in der Anmeldung erwähnt
- THURNER B ET AL: "Generation of large numbers of fully mature and stable dendritic cells from leukapheresis products for clinical application" JOURNAL OF IMMUNOLOGICAL METHODS, ELSEVIER SCIENCE PUBLISHERS B.V.,AMSTERDAM, NL, Bd. 223, Nr. 1, 1. Februar 1999 (1999-02-01), Seiten 1-15, XP004155750 ISSN: 0022-1759
- ROMANI N ET AL: "Generation of mature dendritic cells from human blood An improved method with special regard to clinical applicability" JOURNAL OF IMMUNOLOGICAL METHODS, ELSEVIER SCIENCE PUBLISHERS B.V.,AMSTERDAM, NL, Bd. 196, Nr. 2, 27. September 1996 (1996-09-27), Seiten 137-151, XP004021269 ISSN: 0022-1759

### SEQUENZPROTOKOLL

<110> Schuler, Gerold
<120> Verfahren zur Herstellung gebrauchsfertiger, antigenbeladener oder -unbeladener, kryokonservierter, reifer dendritischer Zellen
<130> 010589wo/JH/ml
<140>
   <141>
<160> 27
<170> PatentIn Ver. 2.1
<210> 1
   <211> 9
   <212> PRT
   <213> Influenza virus
<400> 1
   Gly Ile Leu Gly Phe Val Phe Thr Leu
   1               5
<210> 2
   <211> 10
   <212> PRT
   <213> Künstliche Sequenz
<220>
   <223> Martl/MelanA-analoges Peptid (AS 26-35)
<400> 2
   Glu Leu Ala Gly Ile Gly Ile Leu Thr Val
   1               5                   10
<210> 3
   <211> 10
   <212> PRT
   <213> Influenza virus
<400> 3
   Lys Leu Gly Glu Phe Tyr Asn Gln Met Met
   1               5                   10
<210> 4
   <211> 9
   <212> PRT
   <213> Homo sapiens
<400> 4
   Phe Leu Trp Gly Pro Arg Ala Leu Val
   1 5
<210> 5
   <211> 9
   <212> PRT
   <213> Künstliche Sequenz
<220>
   <223> Beschreibung der künstlichen Sequenz:gp100-analoges Peptid, AS 209-217
<400> 5
   Ile Met Asp Gln Val Pro Phe Ser Val
   1 5
<210> 6
   <211> 9
   <212> PRT
   <213> Homo sapiens
<400> 6
   Tyr Met Asp Gly Thr Met Ser Gln Val
   1 5
<210> 7
   <211> 10
   <212> PRT
   <213> Homo sapiens
<400> 7
   Val Leu Pro Asp Val Phe Ile Arg Cys Val
   1 5 10
<210> 8
   <211> 9
   <212> PRT
   <213> Homo sapiens
<400> 8
   Gly Leu Tyr Asp Gly Met Glu His Leu
   1 5
<210> 9
   <211> 10
   <212> PRT
   <213> Homo sapiens
<400> 9
   Gly Val Tyr Asp Gly Arg Glu His Thr Val
   1 5 10
<210> 10
   <211> 9
   <212> PRT
   <213> Influenza virus
<400> 10
   Cys Thr Glu Leu Lys Leu Ser Asp Tyr
   1 5
<210> 11
   <211> 9
   <212> PRT
   <213> Influenza virus
<400> 11
   Val Ser Asp Gly Gly Pro Asn Leu Tyr
   1 5
<210> 12
   <211> 9
   <212> PRT
   <213> Homo sapiens
<400> 12
   Glu Val Asp Pro Ile Gly His Leu Tyr
   1 5
<210> 13
   <211> 9
   <212> PRT
   <213> Homo sapiens
<400> 13
   Glu Ala Asp Pro Thr Gly His Ser Tyr
   1 5
<210> 14
   <211> 9
   <212> PRT
   <213> Künstliche Sequenz
<220>
   <223> Beschreibung der künstlichen Sequenz:Tyrosinase-analoges Peptid, AS 243-251
<400> 14
   Lys Ser Asp Ile Cys Thr Asp Glu Tyr
   1 5
<210> 15
   <211> 9
   <212> PRT
   <213> Influenza virus
<400> 15
   Ile Leu Arg Gly Ser Val Ala His Lys
   1 5
<210> 16
   <211> 9
   <212> PRT
   <213> Homo sapiens
<400> 16
   Ser Leu Phe Arg Ala Val Ile Thr Lys
   1 5
<210> 17
   <211> 9
   <212> PRT
   <213> Homo sapiens
<400> 17
   Leu Ile Tyr Arg Arg Arg Leu Met Lys
   1 5
<210> 18
   <211> 9
   <212> PRT
   <213> Homo sapiens
<400> 18
   Ala Phe Leu Pro Trp His Arg Leu Phe
   1 5
<210> 19
   <211> 9
   <212> PRT
   <213> Homo sapiens
<400> 19
   Asn Tyr Lys His Cys Phe Pro Glu Ile
   1 5
<210> 20
   <211> 9
   <212> PRT
   <213> Homo sapiens
<400> 20
   Val Tyr Phe Phe Leu Pro Asp His Leu
   1 5
<210> 21
   <211> 9
   <212> PRT
   <213> Homo sapiens
<400> 21
   Ile Met Pro Lys Ala Gly Leu Leu Ile
   1 5
<210> 22
   <211> 9
   <212> PRT
   <213> Homo sapiens
<400> 22
   Ser Glu Ile Trp Arg Asp Ile Asp Phe
   1 5
<210> 23
   <211> 10
   <212> PRT
   <213> Homo sapiens
<400> 23
   Met Glu Val Asp Pro Ile Gly His Leu Tyr
   1 5 10
<210> 24
   <211> 13
   <212> PRT
   <213> Künstliche Sequenz
<220>
   <223> Beschreibung der künstlichen Sequenz:Tyrosinase-analoges Peptid, AS 450-462
<400> 24
   Ser Tyr Leu Gln Asp Ser Val Pro Asp Ser Phe Gln Asp
   1 5 10
<210> 25
   <211> 16
   <212> PRT
   <213> Homo sapiens
<400> 25
   Trp Asn Arg Gln Leu Tyr Pro Glu Trp Thr Glu Ala Gln Arg Leu Asp
   1 5 10 15
<210> 26
   <211> 14
   <212> PRT
   <213> Homo sapiens
<400> 26
   Leu Leu Lys Tyr Arg Ala Arg Glu Pro Val Thr Lys Ala Glu
   1 5 10
<210> 27
   <211> 16
   <212> PRT
   <213> Homo sapiens
<400> 27
   Lys Lys Leu Leu Thr Gln His Phe Val Gln Glu Asn Tyr Leu Glu Tyr
   1 5 10 15

## Beschreibung

Die vorliegende Erfindung betrifft ein Verfahren zur Herstellung kryokonservierter, reifer dendritischer Zellen, wobei unreife dendritische Zellen in Gegenwart von geeigneten Reifungsstimuli kultiviert und die auf diese Weise erhaltenen reifen dendritischen Zellen eingefroren werden. Vor oder nach dem Einfrieren können die dendritischen Zellen mit Antigen beladen werden.

### Hintergrund der Erfindung

Dendritische Zellen (nachfolgend kurz "DC" oder auch "DZ") sind antigenpräsentierende Zellen, die das Immunsystem durch Wechselwirkung mit Lymphozyten beeinflussen. Die meisten DC weisen immunstimulatorische Aktivität auf. Diese klassischen DC können auf verschiedene Weisen *in vivo* die Bildung von Helfer- und Killer-T-Zellen induzieren ("nature's adjuvant"). Insbesondere haben unreife DC, die in peripheren Geweben vorkommen, die Fähigkeit, Antigene zu binden und daraus immunogene MHC-Peptid-Komplexe herzustellen ("antigen processing mode"). Auf reifungsinduzierende Stimuli wie z. B. inflammatorische Cytokine hin entwickeln sich diese unreifen DC durch eine erhöhte Bildung von Adhäsions- und costimulatorischen Molekülen in potente T-Zell-Stimulatoren ("T-cell stimulatory mode"). Gleichzeitig wandern die Zellen in sekundäre lymphatische Organe, um seltene Antigen-spezifische T-Zellen auszuwählen und zu stimulieren. Es konnte gezeigt werden, dass DC, die aus Gewebe oder Blut isoliert wurden und in vitro mit Antigen beladen wurden, nach Rückinjektion als reife DC in vivo immunogen waren. Kürzlich konnte gezeigt werden, dass DC CD4+ und CD8+ T-Zellimmunität sowohl in gesunden Menschen als auch in Krebspatienten induzieren können. Bei immunkompetenten, gesunden Personen konnte eine einzige Auffrischungsinjektion mit reifen DC nicht nur die Häufigkeit, sondern auch die funktionelle Avidität der CD8+ T-Zellantwort verstärken. Aus diesen Gründen sind (insbesondere reife) DC gegenwärtig äußerst vielversprechende-Adjuvantien, um im Menschen potente T-Zellantworten gegen Tumoren und Infektionen hervorzurufen.

Eine Voraussetzung für die Verwendung von DC in der Immuntherapie ist die Entwicklung von Techniken, die es erlauben, eine große Anzahl von DC in Kultur zu produzieren, entweder aus proliferierenden CD34+-Vorläuferzellen oder nicht oder wenig proliferierenden CD14+-monozytischen Vorläuferzellen. Von Monozyten abgeleitete DC werden gegenwärtig häufig verwendet, da sie einfach ohne jede Cytokinvorbehandlung des Spenders hergestellt werden können, und da die resultierende DC-Population recht homogen und am besten charakterisiert ist. Es können z. B. aus adhärenten Monozyten in Abwesenheit von fötalem Kälberserum (FCS) unreife DC hergestellt werden während einer in der Regel sechs- bis siebentägigen Kultur in (GM-CSF + IL-4), gefolgt von einer zumeist ein- bis dreitätigen Reifung, induziert durch autologes Monozyten-konditioniertes Medium. Eine effektive Cryokonservierung dendritischer Zellen oder ihrer Vorläuferzellen hat sich als äußerst schwierig erwiesen, außer in Gegenwart von FCS (Taylor et al. (1990) Cryobiology 27, 269; Makino et al. (1997) Scand. J. Immunol. 45, 618). Da jedoch FCS bei Vakzinierungen nicht zugegen sein darf, war es bislang immer noch notwendig, für jede DC-Vakzinierung DC neu herzustellen, entweder aus frischem Blut, aus frischen Leukaphereseprodukten oder aus gefrorenen PBMC ("peripheral blood mononuclear cells")-Aliquots aus Leukaphereseprodukten (Thurner et al. (1999) J. Immunol. Methods 223, 1). Gefrorene PBMC haben auch den Nachteil, dass nach dem Auftauen erst eine mehrtägige Kultivierung der Zellen erfolgen muß, um sie in DC zu differenzieren. Lewalle et al. (2000) J. Immunol. Methods 240, 69-78 offenbaren ein Verfahren zum Einfrieren unreifer DC, es werden aber nur schlechte Ausbeuten an überlebenden Zellen erhalten (S. 71, linke Spalte oben). Das Einfrieren von mittels GM-CSF und IL-4 aus Monozyten hergestellten unreifen dendritischen Zellen ist auch in der WO 99/46984 offenbart.

Eine Aufgabe der vorliegenden Erfindung ist es daher, eine Zusammensetzung wie z. B. ein Impfstoff bereitzustellen, der reife DC enthält, über einen längeren Zeitraum ohne wesentlichen Aktivitätsverlust aufbewahrt werden kann und bei Bedarf innerhalb kurzer Zeit verabreicht werden kann.

Die erfindungsgemäß eingesetzten DC stammen bevorzugt von demjenigen Patienten, der mit dem Impfstoff behandelt wird (autolog). Alternativ hierzu können die DC aber auch von anderen Patienten stammen (allogen), z. B. aus Leukapheresaten von normalen Freiwilligen, wie z. B. in Thurner et al. (1999) J. Immunol. Methods 283, 1-15 beschrieben.

Überraschenderweise wurde gefunden, dass nur voll ausgereifte DC (nachfolgend auch kurz "reife DC"), die durch Kultivieren in Gegenwart eines geeigneten "Reifungscocktails" mit spezifischen Reifungsstimuli erhältlich sind, nach Einfrieren ohne FCS und Auftauen zu einem hohen Prozentsatz überleben und immunstimulierende Aktivitäten aufweisen, die vergleichbar sind mit denen von frisch präparierten, reifen DC. Beim Kultivieren mit dem Reifungscocktail werden die unreifen DC zu reifen DC differenziert. Ein besonders bevorzugter Reifungscocktail enthält Interleukin-1β (IL-1β), Interleukin-6 (IL-6), Prostaglandin E₂ (PGE₂) und "tumor necrosis factor α" TNFα.

Die vorliegende Erfindung betrifft somit ein Verfahren zur Herstellung kryokonservierter, reifer dendritischer Zellen, die nach Auftauen sofort verfügbar sind, umfassend
a) Bereitstellen von unreifen dendritische Zellen (DC) ;
b) Kultivieren der unreifen DC in einem Kulturmedium, das einen Reifungscocktail mit einem oder mehreren Reifungsstimuli enthält, unter Erhalt von reifen DC;
c) Einfrieren der reifen DC in einem Einfriermedium, das autologes Serum oder autologes Plasma, 5 bis 25% (v/v) DMSO und 2 bis 30% (w/v) einer oder mehrerer Polyolverbindungen enthält.
Das vorstehend definierte Verfahren ist insbesondere zur Herstellung eines Impfstoffs aus reifen, kryokonservierten DC geeignet.

In den Verfahren der Erfindung werden zunächst unreife DC kultiviert, wobei ein geeigneter Reifungscocktail mit einem oder mehreren Reifungsstimuli dem Kulturmedium zugesetzt wird. Geeignete Reifungsstimuli sind dabei IL-1 wie z. B. IL-1β, IL-6, TNF-α, Prostglandine wie z. B. PGE_{2,} IFN-α, Lipopolysacchariden und andere bakterielle Zellprodukte wie z. B. MPL (Monophosphoryllipid A), Lipoteichonsäure, Phosphorylcholin, Calciumionophore, Phorbolester wie PMA, Heat-Schock-Proteine, Nukleotide wie z. B. ATP, Lipopeptide, künstliche Liganden für Toll-ähnliche Rezeptoren, doppelsträngige RNA wie z. B. Poly-I:C, immunstimulatorische DNA-Sequenzen, CD40-Ligand. Besonders bevorzugt im Sinne der Erfindung ist, wenn das Kulturmedium oder der Reifungscocktail IL-1β, IL-6, PGE₂ und TNFα enthält oder der Reifungscocktail Monocyten-konditioniertes Medium (MCM) oder MCM mit zusätzlichem PGE₂ ist. In einer bevorzugten Ausführungsform der Verfahren können die DC während oder nach dem Kultivierungsschritt mit Antigen beladen werden. Nach Reifung und gegebenenfalls Beladung mit Antigen werden die DC in Einfriermedium eingefroren, das autologes Serum oder autologes Plasma (d. h kein heterologes Serum wie z. B. FCS) enthält.

Unreife DC im Sinne dieser Anmeldung sind CD83 (und/oder p55/fascin- und/oder DC-LAMP-) negative (oder nur schwach und/oder zu geringem Prozentsatz positive), im Vergleich zu reifen DC nur geringe Mengen an Klasse-I- und Klasse-II-MHC sowie Adhäsions- bzw. kostimulatorische Moleküle (insbesondere CD86, CD80, CD40) exprimierende Leukozyten, welche nach einem geeigneten Reifungsstimulus in reife DC differenzieren.

Reife DC im Sinne dieser Anmeldung sind Leukozyten, welche sich nach Einwirkung eines Reifungsstimulus aus unreifen DC entwickeln, eine verstärkte Expression von CD83 (und/oder p55/fascin und/oder DC-LAMP) sowie von Klasse-II-und Klasse-I-MHC-Molekülen und Adhäsions- bzw. kostimulatorischen Molekülen zeigen, insbesondere von CD86, CD80, CD40. Weiter zeigen die reifen DC eine deutlich erhöhte T-Zell-stimulatorische Kapazität im Vergleich zu unreifen DC (z. B. zeigen sie im Gegensatz zu unreifen DC eine eindeutige stimulatorische Aktivität in der allogenen MLR noch bei einem DC:T-Verhältnis um ≤ 1:100); zudem ist ein Charakteristikum der reifen DC im Sinne dieser Anmeldung, dass diese DC stabil sind, d. h. ihre Eigenschaften eines reifen Phänotyps und einer starken T-Zell-stimulatorischen Kapazität auch behalten, wenn sie in Abwesenheit von Zytokinen für 1-2 Tage oder länger kultiviert werden. Im Gegensatz dazu sind DC, die nicht voll ausgereift sind, nicht stabil und differenzieren zu unreifen DC oder z. B. zu adhärenten Makrophagen.

Die unreifen DC können aus verschiedenen bekannten Quellen bereitgestellt werden. Die Vorläuferzellen der unreifen DC sind in der Regel nicht-proliferierende CD14-positive mononukleäre Zellen (PBMC; Monozyten) oder proliferierende CD34-positive Zellen. Beispielsweise können aus Leukaphereseprodukten PBMC isoliert werden. Die PBMC können wie beschrieben zu unreifen DC differenziert werden (Thurner et al. (1999) J. Exp. Med. 190, 1669). Dazu werden die PBMC in Gegenwart von IL-4 (oder IL-13; Romani et al. (1996) J. Immunol. Methods 196, 137-151) und GM-CSF ("granulocyte-macrophage-colony stimulating factor") kultiviert. Aus CD14-positiven Monocyten können unreife DC auch durch Kultivierung in Gegenwart von GM-CSF und IFNα hergestellte werden (Santini et al. (2000) J. Exp. Med. 191, 1777-1788). Aus CD34-positiven Zellen können durch Kultivierung in Gegenwart von GM-CSF, SCF ("stem cell factor") und TNFα un reife DC erhalten werden. Die unreifen dendritischen Zellen können auch direkt aus frischem Blut gewonnen werden wie z. B. in Nestle et al. (1998) Nat. Med. 4, 328 beschrieben. Im Blut existieren auch präformierte CD11c+ und CD11c- DC (O'Doherty et al. (1994) Immunology 82, 487-493) sowie M-DC8-DC (Schakel et al. (1999) Pathobiology 67, 287-290). Auch diese DC können aus dem Blut isoliert werden und weiter in dem erfindungsgemäßen Verfahren verwendet werden.

Die bevorzugten Konzentrationen der verschiedenen Reifungsstimuli im Kulturmedium liegen im Bereich von 0,1 ng/ml bis 100 µg/ml, vorzugsweise 1 ng/ml bis 10 µg/ml. Für den besonders bevorzugten Reifungscocktail der vorliegenden Erfindung beträgt die Konzentration der Reifungsstimuli 0,1 bis 100 ng/ml IL-1β, 0,1 bis 100 ng/ml IL-6, 0,1 bis 10 µg/ml PGE₂ und 0,1 bis 100 ng/ml TNFα (am bevorzugtesten ist die Konzentration dieser speziellen Reifungsstimuli ungefähr 10 ng/ml IL-1β, 10 ng/ml IL-6, 1 µg/ml PGE₂ und 10 ng/ml TNFα). Die Konzentrationen beziehen sich auf die Endkonzentrationen der Stoffe im Zellkulturmedium, in dem die DC kultiviert werden. Eine Möglichkeit, die DC in Gegenwart der genannten Wirkstoffe auszureifen, besteht darin, die unreifen DC in Gegenwart von Monocyten-konditioniertem Medium (MCM) zu kultivieren. MCM enthält IL-1β, IL-6, PGE₂ und TNFα. Es kann durch Kultivierung von PBMC in Medium ohne Cytokine gewonnen werden, wie zum Beispiel in Romani et al. (1996) J. Immunol. Methods 196, 137 beschrieben. Bei der Ausreifung der DC durch MCM enthält das Kulturmedium 1-100 %, vorzugsweise 5-25 % MCM. In einer weiteren Ausführungsform der Erfindung erfolgt die Ausreifung der DC durch Zusatz von MCM und einer definierten Menge PGE₂. Es wurde nämlich gefunden, dass Schwankungen in der Fähigkeit von MCM, DC bestmöglich auszureifen, durch Zusatz von PGE₂ (bevorzugt in den oben angegebenen Konzentrationen) aufgefangen werden können. Erfindungsgemäß ist es aber bevorzugt, die DC durch Zusatz definierter optimaler Mengen der Wirkstoffe IL-1β, IL-6, PGE₂ und TNFα auszureifen. Das bedeutet, dass die Reifungszusammensetzung aus gereinigten Zubereitungen der einzelnen Wirkstoffe hergestellt wird. Dadurch werden bessere Ergebnisse erzielt als bei der Ausreifung mit MCM oder MCM+PGE₂. IL-1β, IL-6, PGE₂ und TNFα sind in GMP ("good manufacturing practice")-Qualität erhältlich. Üblicherweise erfolgt die Reifung durch Kultivierung in Gegenwart der genannten Stoffe für wenigstens 1 Stunde, vorzugsweise 2 Stunden und besonders bevor zugt wenigstens 6 Stunden. Besonders bevorzugt im Sinne der vorliegenden Erfindung sind Reifezeiten von 6 bis 96 Stunden, vorzugsweise von 18 bis 36 Stunden (im Falle der Verwendung von Leukopharesen als Ausgangsmaterial) bzw. für 36 bis 60 Stunden (bei Verwendung von Frischblut oder buffy als Ausgangsmaterial).

Erfindungsgemäß werden die DC während und/oder nach dem genannten Reifungsschritt mit wenigstens einem Antigen oder einem Antigen-Antikörperkomplex beladen. Erfindungsgemäß kann die Antigen-Beladung vor oder nach dem Einfrieren erfolgen. Erfolgt sie nach dem Einfrieren, dann werden die DC erst nach dem Wiederauftauen mit Antigen beladen. Bevorzugt ist aber eine Beladung mit Antigen vor dem Einfrieren der DC. Das hat den Vorteil, dass die DC unmittelbar nach dem Auftauen einsatzbereit sind.

Antigene im Sinne dieser Anmeldung sind Proteine, Proteinfragmente, Peptide sowie davon abgeleitete Moleküle (z. B. glykosierte Verbindungen oder Verbindungen mit sonstigen chemischen Modifikationen), aber auch die dafür kodierende Nukleinsäure, Viren, ganze pro- bzw. eukaryontische Zellen oder Fragmente davon oder apoptotische Zellen. Unter "Beladung durch Antigen" wird ein Prozess verstanden, der dazu führt, dass MHC-Moleküle auf der Zelloberfläche der DC Peptide "präsentieren", also einen Komplex mit den MHC-Molekülen aufweisen. Während durch spezifische Peptide eine direkte Beladung der MHC-Moleküle möglich ist, müssen Protein(fragment)e erst prozessiert werden, also zunächst von der Zelle aufgenommen werden. Nach intrazellulärer Spaltung der Proteine und Beladung von MHC mit Peptid werden MHCII-Peptid-Komplexe auf der Zelloberfläche präsentiert. Als Antigene kommen zunächst vor allem Proteine oder Proteinfragmente in Frage. Die Proteine können nativen Ursprungs sein oder rekombinant hergestellt worden sein. Durch die Reifung und Beladung mit Protein-Antigen entstehen auf der Zelloberfläche der DC MHCII-Peptid-Komplexe, die Peptidfragmente des zugesetzten Antigens umfassen. Die Konzentration des Protein-Antigens im Kulturmedium ist in der Regel 0,1 bis 100 µg/ml, vorzugsweise 1 bis 50 µg/ml, am bevorzugtesten 1 bis 10 µg/ml.

Wird als Antigen ein Protein (d. h. ein Polypeptid mit mehr als 50 Aminosäureresten) eingesetzt, so sollte die Beladung vorzugsweise während der Reifung der DC erfolgen. Dann erfolgt eine besonders effektive Präsentation der Antigenfragmente durch MHCII-Moleküle. Das Protein(fragment) muß nicht während des gesamten Reifungszeitraums anwesend sein, es sollte aber wenigstens für einen Teil des Reifungszeitraums sowohl die Reifungszusammensetzung als auch das Protein(fragment) gleichzeitig anwesend sein. Beispiele für Proteinantigene sind KLH (Keyhole Limpet Hemocyanin) als weit verwendetes positives Kontrollantigen, MAGE-3-Protein als Beispiel für ein Tumorantigen, sowie Hepatitis B Oberflächenantigen (HbsAg) oder HIV-1 gp 160-Protein als Beispiel für ein zur Behandlung von viralen Erkrankungen sinnvolles Protein.

Bei einer Beladung mit "kurzen Polypeptiden" oder Proteinfragmenten (z. B. kurze Polypeptide mit bis zu 50 Aminosäureresten, die exakt in die MHC-Moleküle an der DC-Oberfläche hineinpassen) sind neben der obengenannten Beladung während der Reifung auch eine Beladung nach der Reifung, nämlich vor dem Einfrieren oder nach dem Wiederauftauen, möglich. Bei solch einer Beladung nach der Reifung werden die kurzen Polypeptide zu gewaschenen DC zugegeben.

Eine weitere Möglichkeit der Beladung mit Antigen stellt die Zugabe von apoptotischen oder lysierten Zellen zum Kulturmedium dar. Die zugesetzten Zellen können dann von den DC phagocytiert werden. Diese Methode hat den Vorteil, dass neben MHC-Klasse-II-Molekülen auch Klasse-I-Moleküle effektiv beladen werden. Es wurde beispielsweise gezeigt, dass selbst bei Gabe von Proteinen (insbesondere partikulärer Natur) Präsentation auch auf MHC -Klasse-I-Molekülen stattfindet. Beispielsweise kann man, anstatt die DC in Gegenwart von Mage-3 Tumorprotein oder -peptid zu kultivieren, den DC Zellfragmente (von nekrotischen Zellen oder apoptotischen Tumorzellen) zusetzen, die Mage-3 enthalten.

Es können DC auch dadurch mit Antigen beladen werden, dass DC z. B. mit Tumorzellen fusioniert werden. Eine solche Fusion kann mittels Polyethylenglycol oder Elektroporation erreicht werden.

In einer weiteren Ausführungsform der Erfindung werden die DC dadurch mit Antigen beladen, dass sie transfiziert oder viral infiziert werden. Dadurch werden Nukleinsäuren, die für Antigene kodieren, in die DC eingebracht und zur Expression gebracht. Beispielsweise kann eine DNA- oder RNA-Transfektion oder eine Infektion mit Adenoviren in geeigneter Weise durchgeführt werden. Dadurch ist auch eine Beladung der MHC-Klasse-I-Moleküle möglich. Beispielsweise kann auch aus Tumorzellen präparierte RNA transfiziert werden. Zur Transfektion können gängige Methoden wie z. B. Elektroporation eingesetzt werden.

Wie vorstehend ausgeführt, kann während des Reifungszeitraums oder nach dem Reifungszeitraum spezifisches Antigen-Peptid (d. h. "kurze Polypeptide") zugesetzt werden, um MHC-Moleküle der Klasse I oder II direkt zu beladen. Das Peptid-Antigen kann vor der Reifungszusammensetzung zu den Zellen gegeben werden, es wird aber vorzugsweise gleichzeitig oder danach zugesetzt. Die Antigenbeladung erfolgt vorzugsweise für wenigstens 10 min, mehr bevorzugt für 1 bis 24 Stunden, am bevorzugtesten für 3 bis 12 Stunden.

Die Peptide (d. h. "kurze Polypeptide") haben in der Regel wenigstens 8 Aminosäuren. Vorzugsweise haben solche Peptide 8 bis 12 Aminosäuren (MHCI) bzw. 8 bis 25 Aminosäuren (MHCII). Die Konzentration des Peptids im Kulturmedium zur Beladung beträgt in der Regel 0,01 bis 1000 µM, vorzugsweise 0,1 bis 100 µM, am bevorzugtesten 1 bis 20 µM.

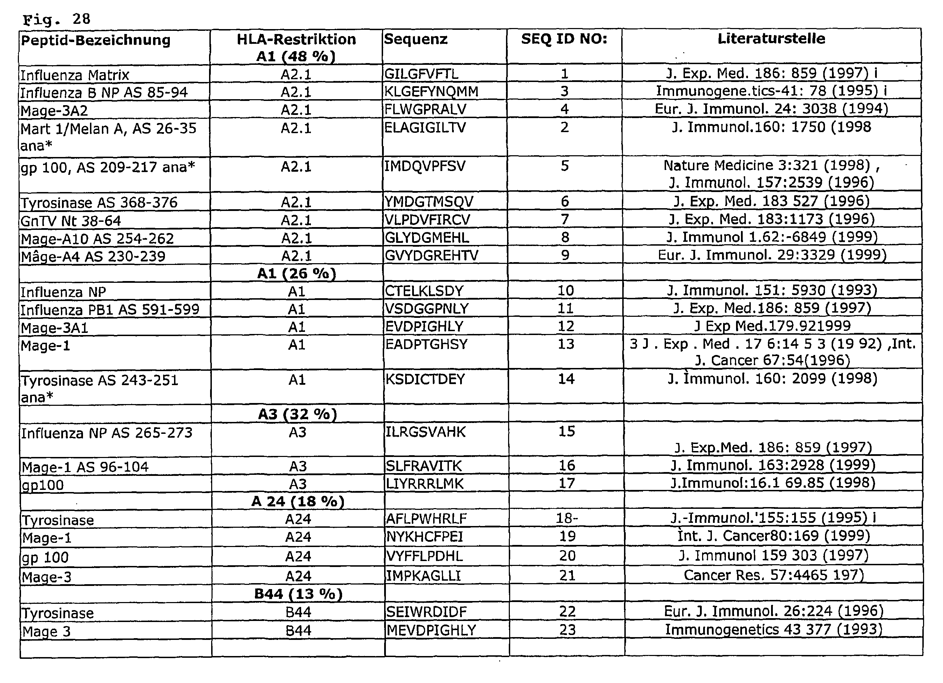

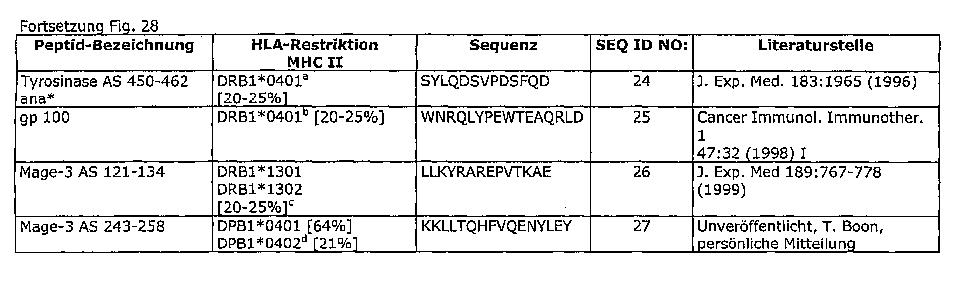

Als einsetzbare Peptide sind alle Peptide denkbar, die von MHC-Molekülen präsentiert werden können. Bevorzugt sind Peptide, die von Proteinen abgeleitet sind, die von Krankheitserregern stammen. Dabei kann es sich auch um Peptide handeln, die Variationen der natürlich vorkommenden Aminosäuresequenz aufweisen. Solche Variationen sind in der Regel ein oder zwei Aminosäureaustausche. Beispiele für Peptidantigene sind das Influenza-Matrix-Peptid (IMP) mit der Aminosäuresequenz GILGFVFTL (SEQ ID NO:1) oder das Melan A-analoge Peptid mit der Sequenz ELAGIGILTV (SEQ ID NO:2). Beispiele für weitere mögliche Peptide sind in Fig. 28 dargestellt.

Neben "Peptid-/Proteinpulsing" kann auch "Peptid-/Protein-transloading" durchgeführt werden, um DC mit Antigen zu beladen.

Wie vorstehend angeführt, können die DC auch mit Antigen-Antikörperkomplexen beladen werden. Geeignete Antigene hierfür sind alle vorstehend erwähnten Antigene. Antigen-Antikörperkomplexe im Sinne der vorliegenden Erfindung sind Komplexe dieser Antigene mit geeigneten Antikörpern, z. B. Antigen-IgG- oder IgE-Komplexe. Das Beladen von DC mit solchen Antigenen ist in Reynault, A. et al., J. Exp. Med. 189(2):371-80 (1999) beschrieben, worauf hier ausdrücklich Bezug genommen wird.

Nach Reifung und gegebenenfalls Antigenbeladung der DC werden die reifen DC in Einfriermedium eingefroren. Das Einfriermedium enthält neben dem autologen Serum oder Plasma noch 5 bis 25 % (v/v)) DMSO und 2 - 30 (w/v)% aus einer oder mehreren Polyolverbindungen. DMSO ist vorzugsweise in einer Konzentration von 10 bis 20 % (v/v), am bevorzugtesten ungefähr 10 % DMSO, im Einfriermedium enthalten. Das Einfriermedium enthält das autologe Serum oder Plasma in einer Konzentration von 2 bis 90 % (w/v), bevorzugt 5 bis 80 % (w/v), besonders bevorzugt in einer Konzentration von 10 bis 30 % (w/v). Die Polyolverbindungen werden insbesondere ausgewählt aus Glukose, Dextran, Sucrose, Ethylenglykol, Erythrit, D-Ribit, D-Mannit, D-Sorbit, Inosit und D-Laktose, wobei eine Konzentration von 5 bis 10 % bevorzugt ist und etwa 5 % (w/v) besonders bevorzugt ist. Das bevorzugteste Einfriermedium ist reines autologes Serum mit ungefähr 10 % DMSO und ungefähr 5 % Glukose. In der Regel werden die Zellen vor dem Einfrieren abzentrifugiert, um sie zu konzentrieren und im Einfriermedium aufzunehmen.

Die bevorzugte Konzentration von Zellen im Einfriermedium ist 1 bis 100 x 10⁶ Zellen/ml, die bevorzugteste Konzentration ist ungefähr 5 x 10⁶ Zellen/ml bis 20 x 10⁶ Zellen/ml.

Es wurde ebenfalls gefunden, dass die Überlebensrate von DC erhöht wird, wenn die DC vor dem Einfrieren oder nach dem Auftauen mit einem anti-apoptotischen Molekül in Kontakt gebracht werden. In einer bevorzugten Ausführungsform des erfindungsgemäßen Verfahrens werden daher vor dem Einfrieren oder nach dem Auftauen die DC mit einem Molekül in Kontakt gebracht, das die Apoptose hemmen kann. Bevorzugte anti-apoptotische Moleküle sind CD40 Ligand (CD40L)(Morris et al. (1999) J. Biol. Chem. 274, 418), TRANCE (Wong et al. (1997) J. Exp. Med. 186, 2075) oder RANKL (Anderson et al. (1997) Nature 390, 175). Vorzugsweise wird wenigstens eines der Moleküle vor dem Einfrieren oder nach dem Auftauen der DC wenigstens 10 min, bevorzugt wenigstens 1 Stunde, bevorzugter wenigstens 4 Stunden dem Kulturmedium zugesetzt. Bevorzugte Konzentrationen im Kulturmedium sind 1 ng/ml bis 5 µg/ml (RANKL/TRANCE) bzw. 1 ng/ml bis 1 µg/ml (CD40L). Besonders bevorzugte Konzentrationen sind 0,5 bis 1 µg/ml (RANKL/TRANCE) und 0,1 bis 0,5 µg/ml (CD40L).

In einer bevorzugten Ausführungsform des Verfahrens werden die DC weiterhin in Gegenwart des immunosuppressiven Interleukin-10 (IL-10) oder anderer Immun-/Reifungsmodulatoren wie z. B. Vitamin D3 und dessen Derivate, Fumarsäure und Derivate davon wie z. B. Ester usw., Mycophenolatmofetil usw. kultiviert. Derartig behandelte Zellen werden nicht zur Stimulation des Immunsystems eingesetzt, vielmehr soll eine Toleranz gegen bestimmte Antigene induziert werden. Die Konzentration dieser Modulatoren und von IL-10 im Medium beträgt 1 - 1000 ng/ml, bevorzugt 10 - 100 ng/ml.

Der mittels dem Verfahren der vorliegenden Erfindung erhältliche Impfstoff kann neben den reifen, Antigen-beladenen DC weiterhin phar mazeutisch verträgliche Hilfsstoffe enthalten. Nach dem Auftauen des Impfstoffs können noch verschiedene Stoffe zugesetzt werden, die pharmazeutisch verträglich und für eine Verabreichung vorteilhaft sind.

Die vorliegende Erfindung stellt zum ersten Mal ein Verfahren zur Verfügung, in dem ein Impfstoff mit reifen, Antigen-beladenen DCs eingefroren werden kann. Ein wesentlicher Vorteil der Erfindung ist es, dass die Überlebensrate der DC nach dem Auftauen sehr hoch ist. Nach dem Auftauen der eingefrorenen DC erhält man in der Regel mehr als 75 %, bevorzugt mehr als 85 % überlebende DC, bezogen auf die Anzahl eingefrorener DC. Derartig hohe Überlebensraten von aufgetauten DC waren bislang ohne Zusatz von FCS nicht erreicht worden. Dies ist aber eine wichtige Voraussetzung, um eine wirksame Immunstimulation zu erzielen. Ein erheblicher Vorteil des Einfrierens von reifen, Antigen-beladenen DC ist, dass zahlreiche Aliquots eines Impfstoffs hergestellt und eingefroren werden können. Dadurch ist der Impfstoff bei Bedarf sofort verfügbar und kann ohne langwierige Kultur-Schritte innerhalb kürzester Zeit appliziert werden. Durch eine Antigenbeladung erst nach Einfrieren und Wiederauftauen reifer DC ist es möglich, die DC nach den jeweiligen Gegebenheiten variabel zu beladen. Beispielsweise kann bei einer sich entwickelnden Überstimulation bzw. Allergie gegenüber einem der zur Vakzination eingesetzten Peptide auf dieses bei der Beladung verzichtet werden.

Es wurde überraschenderweise gefunden, dass es zum Auffinden geeigneter Verfahren zum Einfrieren von DC nicht ausreichend ist, die unmittelbaren Einfrierparameter wie z. B. Einfriermedium und Abkühlgeschwindigkeit zu variieren und die Überlebensrate unmittelbar nach dem Auftauen zu bestimmen. Diese unmittelbare Überlebensrate entspricht nicht unbedingt einem Überleben frisch hergestellter DC während mehrtägiger Kultur in der Abwesenheit von Cytokinen.

Vielmehr spielt auch die Reifung bzw. der verwendete Reifungsstimulus vor dem Einfrieren eine entscheidende Rolle für das Überleben der Zellen. Der Erfindung liegt somit auch die Erkenntnis zugrunde, dass der erfindungsgemäße Reifungsstimulus nicht nur zu einer vollen DC-Reifung führt, sondern auch DC liefert, die besser überleben können als DC, die durch andere Stimuli gereift worden sind. Bisher wurde dem Reifungsstimulus im Hinblick auf das Einfrieren überhaupt keine Beachtung geschenkt.

Es wurde auch gefunden, dass es eine hervorragende Methode zur Optimierung des Verfahrens zum Einfrieren von DC ist, DC mittels verschiedener Reifungsstimuli auszureifen und dann als "Read Out" das Überleben nach Kultur in Komplettmedium ohne jeglichen Cytokinzusatz zu testen. Der Reifungsstimulus, der am höchsten viable DC induziert, wird zur Herstellung von DC verwendet, die dann eingefroren werden.

### Figurenbeschreibung

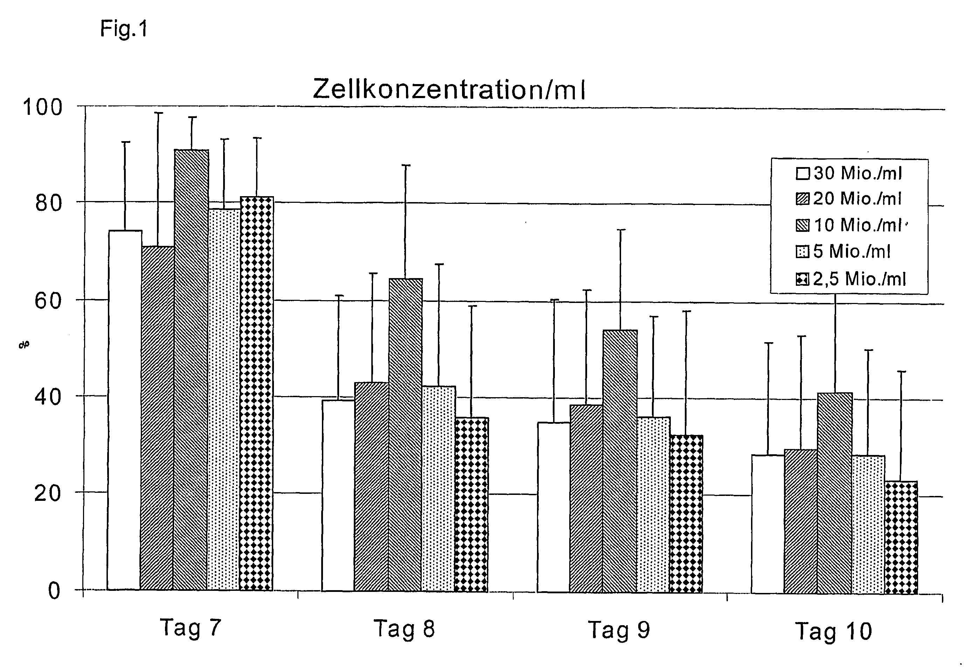

Figur 1 zeigt den Einfluß der Zellkonzentration in den Einfriergefäßen auf die Ausbeute an DC nach dem Auftauen. Reife DC wurden aus Leukaphereseprodukten gesunder Erwachsener hergestellt, wobei zunächst adhärente Monozyten durch sechstägige Kultur in GM-CSF + IL-4 in unreife DC umgewandelt wurden, dann eine Reifung für einen Tag durch einen Vierkomponenten-Cocktail bestehend aus TNFα + IL-1β + IL-6 + PGE₂ erfolgte (s. Beispiel 1). Reife (Tag 7) DC wurden mit einer Einfriergeschwindigkeit von -1°C/min in reinem autologen Serum + 10 % DMSO + 5 % Glukose bei verschiedenen Zelldichten (DC-Zahl/ml) eingefroren. Nach dem Auftauen der reifen DC wurden die Ausbeuten lebender DC (angegeben als Anteil an der Anzahl eingefrorener DC in Prozent) unmittelbar (= d7) und nach mehreren Tagen in Kultur (nach 1 Tag = d8, nach 2 Tagen = d9, etc.) bestimmt. Die letztgenannte Bestimmung wurde in Komplettmedium, aber ohne Zusatz von GM-CSF oder IL-4, durchgeführt, da dieser harte "washout test" eine etablierte Methode ist, um zu bestimmen, ob DC tatsächlich voll ausgereift und stabil sind. Einfrieren bei 10 x 10⁶ reifen DC/ml lieferte die besten Ergebnisse (p < 0,05 an Tag 7, p < 0,01 an allen anderen Tagen; n = 3).
Figur 2 zeigt den Einfluß verschiedener Einfriermedien auf die DC-Ausbeute nach dem Wiederauftauen. DC wurden hergestellt wie in der Legende zu Figur 1 beschrieben, und Aliquots wurden in einer Konzentration von 10 x 10⁶ DC/ml in HSA + 10 % DMSO, in autologem Serum + 10 % DMSO sowie in autologem Serum + 10 % DMSO + 5 % Glukose (Endkonzentrationen) eingefroren. Die DC-Ausbeuten nach Einfrieren und Wiederauftauen wurden bestimmt wie für Figur 1 beschrieben. Einfrieren in autologem Serum + 10 % DMSO + 5 % Glukose ergab die besten Resultate (p < 0,05 an Tag 8; n = 4).

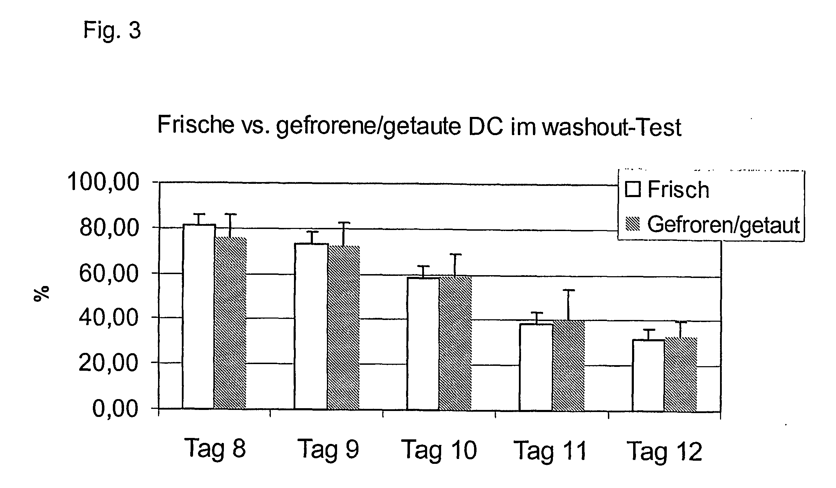

Figur 3 zeigt die Überlebensrate frischer DC im Vergleich mit der eingefrorener und wiederaufgetauter DC in "Washout tests". Reife, d7-DC wurden wie zu Figur 1 beschrieben hergestellt, und frisch hergestellte sowie eingefrorene (-1°C/min in reinem autologen Serum + 10 % DMSO + 5 % Glukose bei einer Zelldichte von 10 x 10⁶ DC/ml) und wiederaufgetaute (nach mehr als 3 Stunden Lagerung in der Gasphase von flüssigem Stickstoff) DC wurden dann für mehrere Tage in Medium ohne Cytokine ("washout test") wie in Figur 1 beschrieben kultiviert. Die Ausbeuten an lebenden DC sind als Prozentangaben der Gesamtzahl an DC, die in die Wells an Tag 7 ausgesät wurden, angegeben. Es gab keinen statistisch signifikanten Unterschied zwischen frisch hergestellten und gefrorenen/aufgetauten DC (n = 4).
Figur 4 zeigt, dass Einfrieren und Wiederauftauen die charakteristische Morphologie reifer DC nicht verändert. Reife, d7-DC, die wie zu Figur 1 beschrieben hergestellt worden waren, wurden für weitere 2 Tage entweder unmittelbar weiterkultiviert oder nach Einfrieren, mindestens 3 Stunden Lagerung in der Gasphase von flüssigem Stickstoff und Wiederauftauen weiterkultiviert. Sogar nach 48 Stunden weiterer Kultur in der Abwesenheit von Cytokinen ("washout test") blieben die nicht eingefrorenen (links) und die eingefrorenen/wiederaufgetauten DC (rechts) stabile, nicht adhärierende Zellen.

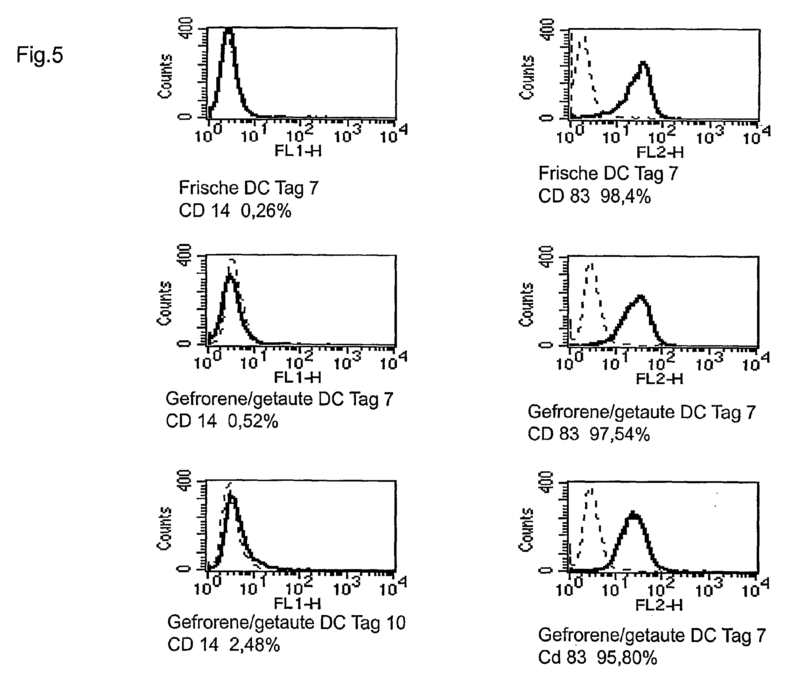

Figur 5 zeigt, dass Einfrieren und Wiederauftauen den charakteristischen Phänotyp reifer DC nicht verändert. Reife, d7-DC, die wie zu Figur 1 beschrieben frisch hergestellt worden waren, wurden durch FACS-Analyse phänotypisiert (frische DC, Tag 7). Aliquots wurden eingefroren, mindestens 3 Stunden in der Gasphase von flüssigem Stickstoff gelagert, wiederaufgetaut, anschließend wurde ihr Phänotyp unmittelbar (DC eingefroren/aufgetaut d7) oder nach weiteren 3 Tagen in Kultur (DC eingefroren/aufgetaut d10) in der Abwesenheit von Cytokinen ("washout test") bestimmt. Eingefrorene/wiederaufgetaute, reife, d7-DC haben den charakteristischen Phänotyp reifer DC (CD14-, CD83 homogen++), sogar nach Entfernen von Cytokinen und weiterer Kultur für 3 Tage behalten.
Figur 6 zeigt, dass Einfrieren und Wiederauftauen die stimulatorische Kapazität reifer DC in der primären allogenen MLR ("Mixed Leukocyte Reaction") nicht verändert. Reife d7-DC wurden wie zu Figur 1 dargestellt hergestellt und die allostimulatorische Potenz frisch hergestellter, nicht eingefrorener DC wurde mit einem Aliquot dieser DC, das eingefroren und wiederaufgetaut worden war (nach 3-stündiger Lagerung in flüssigem Stickstoff), verglichen.

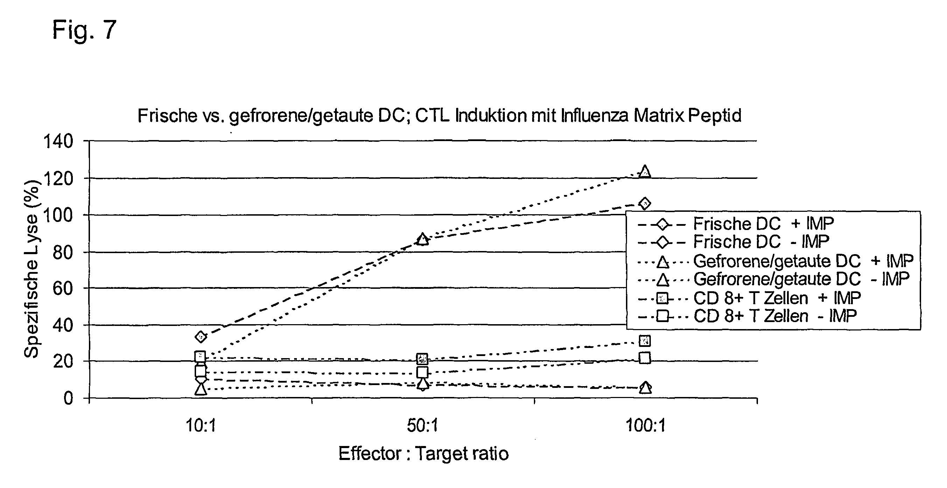

Figur 7 zeigt, dass Einfrieren und Auftauen die Fähigkeit reifer DC, eine starke IMP-spezifische CTL-Antwort zu induzieren, nicht verändert. Frisch hergestellte oder eingefrorene/wiederaufgetaute HLA-A2.1 + reife d7-DC wurden mit Influenza-Matrixpeptid (= IMP) GILGFVFTL (SEQ ID NO:1) beladen oder unbehandelt gelassen und mit autologen CD8⁺-T-Zellen (T:DC-Verhältnis = 10:1) ohne Zugabe jeglicher Cytokine kultiviert. Zusätzlich wurden gereinigte CD8⁺-T-Zellen ohne DC ± Zugabe von IMP kultiviert. Nach 7 Tagen wurden die T-Zellen geerntet und auf ihre zytolytische Aktivität hin untersucht, wozu ein Standard-⁵¹Cr-Freisetzungsassay verwendet wurde. T2-Zellen, die ohne oder mit 10 µg/ml IMP gepulst waren, dienten als Zielzellen.

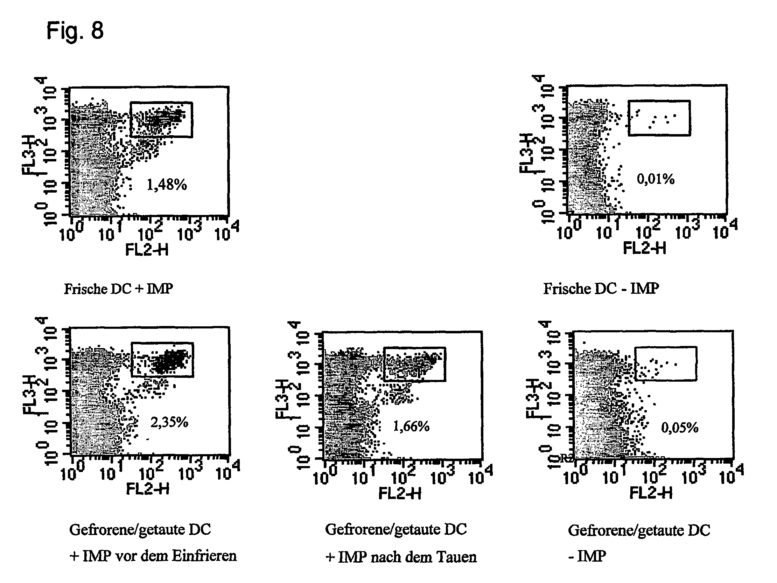

Figur 8 zeigt, dass Einfrieren und Auftauen die Fähigkeit reifer DC, eine starke IMP-spezifische CD8⁺-T-Zellantwort zu induzieren, nicht verändert (HLA-A2.1/Peptidtetrameranalyse). Frisch hergestellte oder eingefrorene/wiederaufgetaute HLA-A2.1 + reife d7-DC wurden mit IMP beladen (im Fall von gefrorenen/wiederaufgetauten Zellen vor dem Einfrieren oder nach dem Auftauen) oder unbehandelt gelassen und für 7 Tage mit autologen, nicht-adhärierenden Fraktionen von PBMC (PBMC:DC-Verhältnis = 30:1) ohne Zugabe jeglicher Cytokine co-kultiviert, geerntet und mit HLA-A2.1/IMP-Tetrameren (X-Achse) und anti-CD8 (Y-Achse) doppelgefärbt. Die Expansion IMP-Peptid-spezifischer CD8⁺-T-Zellen (der Prozentsatz Tetramer-bindender CD8⁺-T-Zellen ist in der Figur angegeben) ist vergleichbar für frische und eingefrorene DC. Beladung von DC vor dem Einfrieren und nach dem Auftauen zeigt keinen Unterschied.

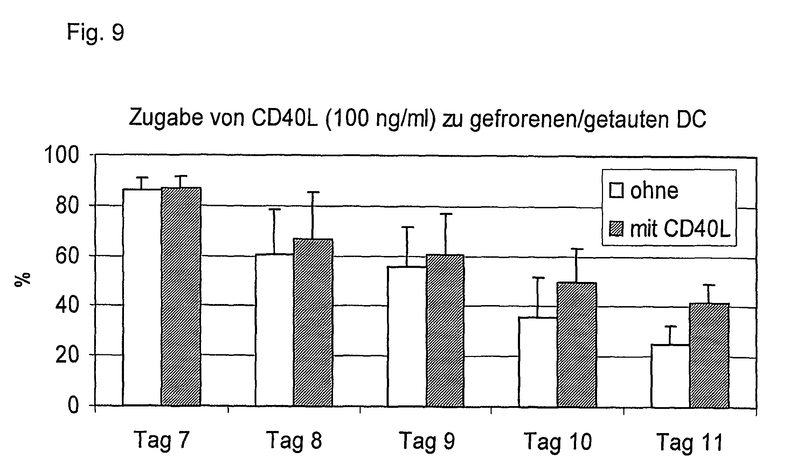

Figur 9 zeigt, dass Kontakt mit CD40L die Überlebensrate aufgetauter DC weiter verbessert. Reife d7-DC wurden hergestellt wie zu Figur 1 beschrieben. Nach dem Auftauen wurde lösliches, primäres CD40L (100 ng/ml) für 4 Stunden zugesetzt, dann wurden die DC gewaschen und für mehrere Tage in Medium ohne Cytokine ("washout test") kultiviert wie in der Legende zu Figur 3 beschrieben. Die Ausbeuten lebender DC (angegeben als Prozentsatz eingefrorener DC) wurden unmittelbar (= d7) sowie nach mehreren Tagen in Kultur (nach 1 Tag = d8, nach 2 Tagen = d9, etc.) bestimmt. CD40L-Behandlung von DC ergab eine verbesserte Überlebensrate, insbesondere über Tag 2 der weiteren Kultur hinaus (p < 0,01 an Tag 10 und Tag 11; n = 5).
Figur 10 zeigt, dass DC erfolgreich mit Protein-Antigen vor dem Einfrieren beladen werden können. DC wurden in ihrem unreifen Stadium durch Zugabe des Modellantigens Tetanustoxoid (TT) (10 µg/ml) an Tag 6 gepulst. Reife DC wurden dann an Tag 7 geerntet und eingefroren wie in der Legende zu Figur 3 beschrieben. Eingefrorene, TT-gepulste DC waren ebenso stimulatorisch wie frisch hergestellte TT-gepulste, reife DC.

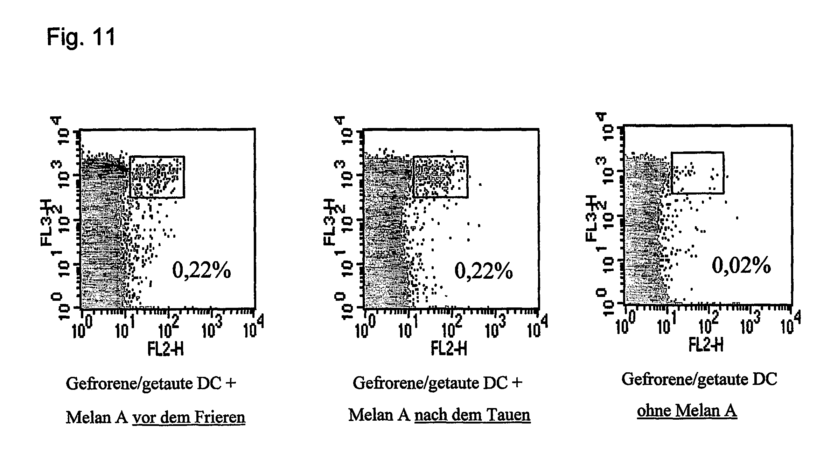

Figur 11 zeigt, dass DC erfolgreich mit Peptid-Antigen vor Einfrieren beladen werden können. Eingefrorene/aufgetaute (siehe Figur 3) HLA-A2.1 + reife d7-DC wurden mit Melan-A-analogem Peptid ELAGIGILTV (SEQ ID NO:2) entweder vor dem Einfrieren oder nach dem Auftauen beladen oder unbehandelt gelassen und für 7 Tage mit autologen, nicht-adhärierenden Fraktionen von PBMC (PBMC:DC-Verhältnis = 20: 1) ohne Zugabe jeglicher Cytokine co-kultiviert, geerntet und mit HLA-A2.1/Melan-A-Tetrameren (X-Achse) und anti-CD8 (Y-Achse) doppelgefärbt. Die Expansion Melan-A-Peptid-spezifischer CD8⁺-T-Zellen (der Prozentanteil Tetramer-bindender CD8⁺-T-Zellen ist in der Figur angegeben) ist vergleichbar für eingefrorene DC, die vor dem Einfrieren oder nach dem Auftauen beladen worden sind. Siehe dazu auch Figur 8.
Figur 12 zeigt die Induktion einer Helferzell Typ 1-Antwort gegen KLH in Patienten, die wie in Beispiel 6 beschrieben vakziniert wurden.
Figuren 13-15 zeigen die Ergebnisse von Beispiel 7.
Figur 16 zeigt eine schematische Darstellung des Versuchsaufbaus von Beispiel 8. Die Ergebnisse des Beispiels 8 sind in Figuren 17 und 18 zusammengefasst.
Figur 17A zeigt die Gesamtzahl der DC nach Beladung mit Tumor-Zell-Lysat oder nekrotischen Tumorzellen *und* nachfolgender Kryokonservierung.
Figur 17B zeigt die Allostimulatorische Potenz von DC nach Beladung mit Tumor-Zell-Lysat oder nekrotischen Tumorzellen und nachfolgender Kryokonservierung.
Figur 17C zeigt das Resultat der FACS Analyse nach Tumorbeladung und Kryokonservierung.
Figur 18A zeigt das Ergebnis der Bestimmung der Gesamtzahl der DC nach Beladung mit apoptotischen Tumorzellen *und* nachfolgender Kryokonservierung.
Figur 18B zeigt die Allostimulatorische Potenz von DC nach Beladung mit apoptotischen MEL 526 Melanomzellen und nachfolgender Kryokonservierung.
Figur 18C zeigt die Ergebnisse der FACS Analyse der MAGE-1/HLA-A1 Ag-Expression auf Mage-1 Peptid-gepulsten DC vor bzw. nach Kryokonservierung.
Figur 18D: zeigt den Vergleich der Expression von MAGE-1/HLA-A1 Komplexen (bestimmt mittels Antikörper gegen MAGE-1/HLA-A1 Komplex) vor und nach Kryokonservierung auf DC, welche auf verschiedene Art beladen wurden.

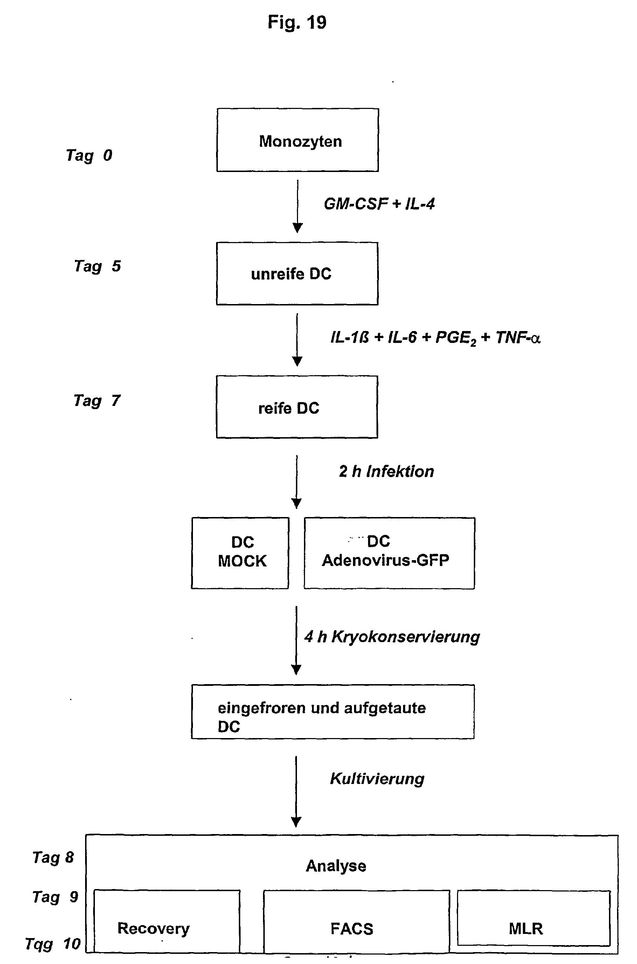

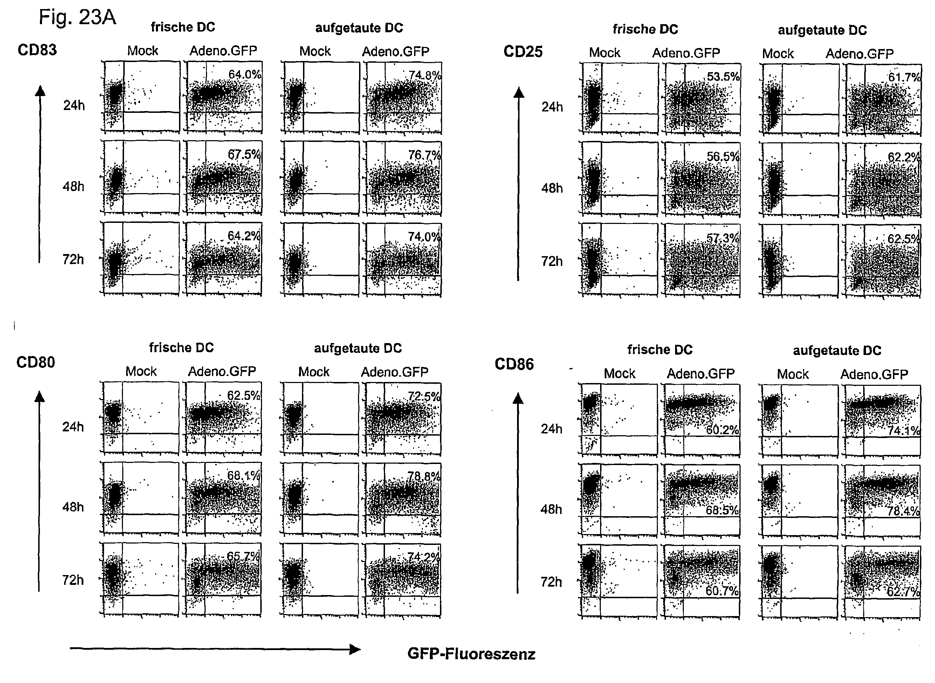

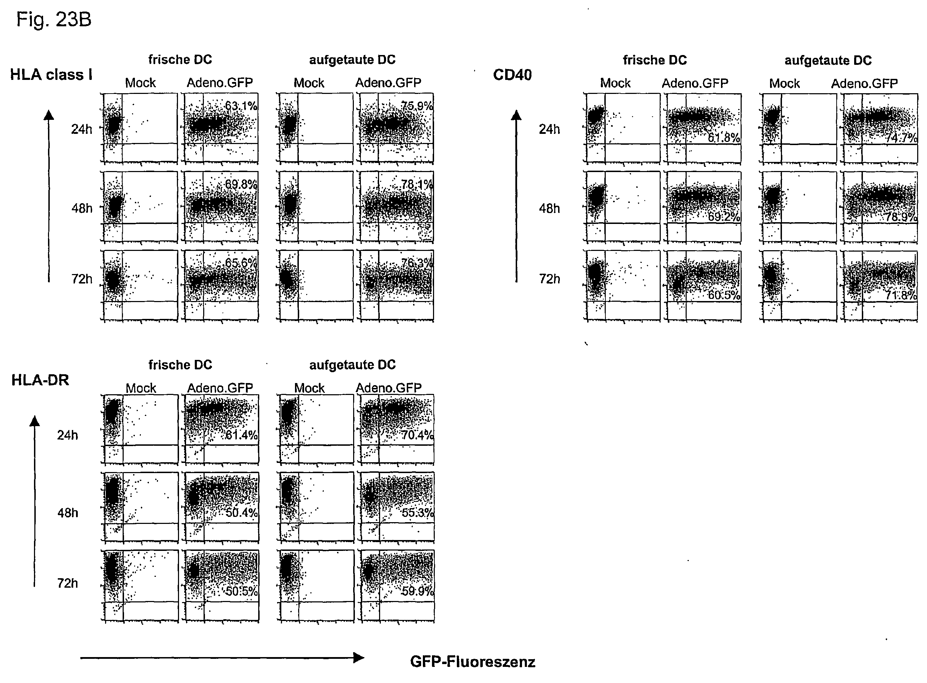

Figur 19 zeigt eine schematische Darstellung des Versuchsaufbaus von Beispiel 9, in dem Adenovirus infizierte dendritische Zellen mit oder ohne Kryokonservierung verglichen werden. Die Ergebnisse des Beispiels 9 sind in Figuren 20 bis 23 zusammengefasst.
Figur 20 zeigt die Wiedergewinnung von Adenovirus infizierten dendritischen Zellen nach Kryokonservierung. Die Wiedergewinnungsrate von adeno-GFP infizierten DC nach Einfrieren und Wiederauftauen versus Mock-treated (nichttransfizierten) DC ist vergleichbar.
Figur 21: CD4-T-Zell stimulierende Aktivität von Adenovirus-infizierten dendritischen Zellen nach Kryokonservieren. Es kann gezeigt werden, dass die Kryokonservierung die allostimulierende Aktivität von Adenovirus-infizierten DC nicht verändert.
Figuren 22A und 22B zeigen, dass ein ähnlicher Phenotyp bei Adenovirus-infizierten DC mit oder ohne Kryokonservierung vorliegt.
Figuren 23A und 23B zeigen einen Phenotypen von Adenovirus-infizierten DC mit oder ohne Kryokonservierung.

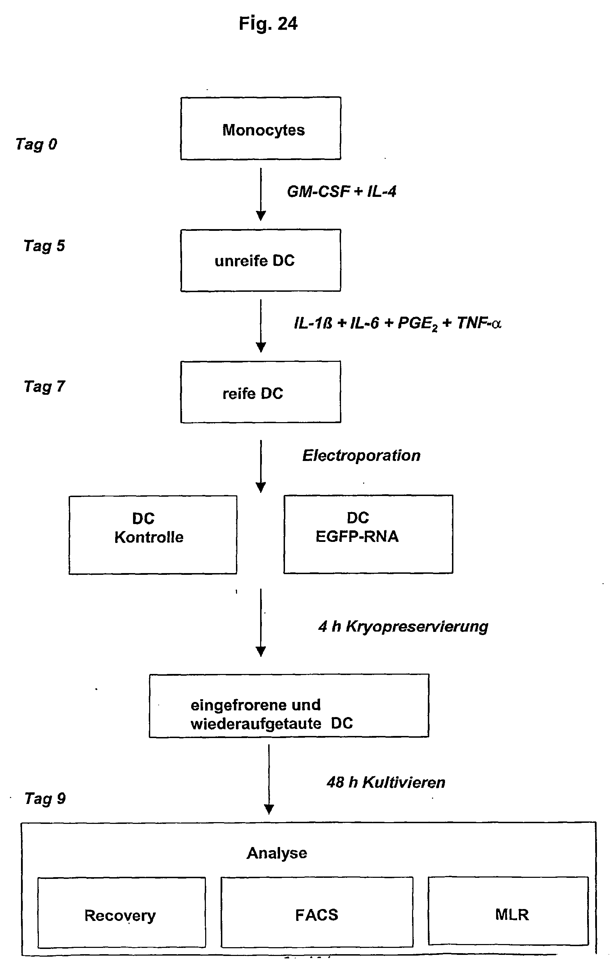

Figur 24 zeigt den schematischen Versuchsablauf von Beispiel 10 im Vergleich zu RNA-elektroporierten DC mit oder ohne Kryokonservierung. Die Ergebnisse sind in Figuren 25 bis 27 zusammengefasst.
Figur 25 zeigt, dass die Wiedergewinnung nach Kryokonservierung von EGFP-RNA-elektroporierter DC ähnlich ist wie in dem Kontrollexperiment.
Figur 26 zeigt, dass die Kryokonservierung die allostimulatorische Kapazität der EGFP-RNA-transfizierten DC nicht ändert.
Figur 27 zeigt, dass der Phenotyp von RNA-transfizierten DC ähnlich ist wie in dem Kontrollexperiment.
Figur 28 zeigt MHC-Klasse I- und II-restringierte Melanom- und Influenzaviruspeptide, die in dem Verfahren der vorliegenden Erfindung eingesetzt werden können.
   Abkürzungen: AS = Aminosäuren, NT = Nukleotide, NP = Nukleoprotein, ana* = analoges Peptid
   ^{a} häufigster DR4-Subtyp, ungefähr 80 %
   ^{b} häufigster DR4-Subtyp, ungefähr 80 %
   ^{c} Bis heute wurden 30 verschiedene HLA-DR13-Allele beschrieben. Es wurde Restriktion für DRB!*1301 und DRB1*1302 gezeigt, die zusammen 80 % der DR13-Allele ausmachen. Es ist möglich, dass auch andere DR13-Allele das Peptid präsentieren.
   ^{d} Das Epitop wird durch dieses zweite HLA-DP4-Allel weniger effektiv präsentiert als durch das Allel DPB1*0401.

Die folgenden Beispiele sollen die Erfindung näher erläutern, schränken sie jedoch nicht ein.

### Beispiel 1

Es wurde die Überlebensrate von frisch hergestellten, unreifen DC und von durch verschiedene Reifungsstimuli gereiften DC bestimmt.

Folgende verschiedene Reifungsstimuli wurden getestet:
- Monozyten-konditioniertes Medium, hergestellt und verwendet wie beschrieben (Thurner et al. (1999) J. Immunol. Methods 223, 1)
- 10 ng/ml TNFα
- 10 ng/ml TNFα + 1 µg/ml PGE₂
- 10 ng/ml TNFα + 10 ng/ml IL-1β + 100 U/ml IL-6 + 1 µg/ml PGE₂ ("Reifungscocktail")
- bis zu 1,0 µg/ml LPS von Salmonella abortus equi
- doppelsträngige RNA (Poly I:C, 20 µg/ml)
- 50 - 1000 ng/ml CD40L

Herstellung von aus Monozyten abgeleiteten DC aus PBMC: Als Komplettmedium wurde RPMI 1640 mit 20 µg/ml Gentamicin, 2 mM Glutamin und 1 % hitzeinaktiviertem (56°C, 30 min) autologem humanem Plasma verwendet. Leukaphereseprodukte wurden als Monozyten-Separationsprodukte aus gesunden Zytapheresespendern hergestellt wie beschrieben (Thurner et al. (1999) J. Immunol. Methods 223, 1). Peripheral blood mononuclear cells (PBMC) wurden dann durch Zentrifugation in Lymphoprep isoliert, und DC wurden aus Plastik-adhärierenden Fraktionen der PBMC wie beschrieben hergestellt(Thurner et al. (1999) J. Immunol. Methods 223, 1). Dazu wurden unreife DC durch Kultur in Komplettmedium mit rekombinantem humanem GM-CSF (800 U/ml) und IL-4 (500 U/ml) erhalten. An Tag 6 wurden die verschiedenen, oben genannten Reifungsstimuli zu den DC gegeben, an Tag 7 wurden die reifen DC geerntet und für weitere zwei Tage in Abwesenheit von Cytokinen weiterkultiviert. Die Überlebensraten nach zwei Tagen Kultur in Prozent der ausgesäten DC waren:
38,25 % für unreife DC,
76,2 % für (IL-1β + IL-6 + PGE₂ + TNFα) -gereifte DC,
13,5 % für TNFα-gereifte DC,
37,0 % für (TNFα + PGE₂)-gereifte DC,
31,8 % für (Poly I:C)-gereifte DC und
49,6 % für CD40L-gereifte DC (bei 500 ng/ml).

Die Unterschiede waren statistisch signifikant.

### Beispiel 2

Reife (Tag 7) DC wurden folgendermaßen eingefroren: DC wurden in Cryogefäßen resuspendiert in unterschiedlichen Konzentrationen (5, 20, 40, 60 und 100 x 10⁶ pro ml) entweder in reinem autologem Serum oder in 20 % humanem Serumalbumin (HSA; bestehend aus 1000 ml Elektrolytlösung supplementiert mit 200 g humanen Plasmaproteinen mit wenigstens 95 % Albumin; DRK Blutspendedienst Baden-Württemberg, Baden-Baden, Deutschland). In die entstandene DC-Suspension wurde 1:1 mit den verschiedenen nachstehend beschriebenen Einfrierlösungen gemischt, dann sofort in ein 1,0 oder 1,8 ml Cryogefäß überführt. Unmittelbar danach wurden die Gefäße auf -80°C abgekühlt in einem "Cryo Freezing Container" (Nalgene Cryo 1°C Freezing Container, Abkühlgeschwindigkeit -1°C/min) und schließlich in die Gasphase von flüssigem Stickstoff überführt, wo sie bis zu sieben Monaten aufbewahrt wurden.

### a) Einfriermedien

- HSA + DMSO ± Glukose [bestehend aus 20 % HSA-Lösung (siehe oben + 10, 15, 20 und 25 % (v/v) DMSO ± Glukose (Glukosteril 40 %^{™}, Fresenius, Deutschland, Wirkstoff: Glukosemonohydrat) zu 2, 4, 6, 10, 20 oder 30 % (v/v)];
- Serum + DMSO ± Glukose [reines autologes Serum + 20 % (v/v) DMSO ± Glukose zugesetzt zu 2, 4, 6, 10, 20 oder 30 %];
- Erythrocyte Freezing Solution^{™} [Erythrocyte Freezing Solution^{™} (Fresenius, Dreieich, Deutschland), bestehend aus 38 % Glycerol, 2,9 % Sorbitol und 0,63 % Natriumchlorid in sterilem Wasser];
- Cell Processing Solution^{™} + DMSO (Fresenius, Dreieich, Deutschland, bestehend aus 6 % Hydroxyethylstärke in 0,9 % Natriumchlorid, üblicherweise benutzt zur Sedimentation von Erythrozyten) + 10 oder 15 % (v/v) DMSO.

### b) Auftaubedingungen

Zum Auftauen gefrorener reifer (Tag 7) DC wurden folgende vier Verfahren untersucht:
1. DC wurden in einem 56°C Wasserbad aufgetaut, dann ohne Waschen in 10 bis 20 ml eiskaltes Komplettmedium (supplementiert mit 800 U/ml GM-CSF und 500 U/ml IL-4) in Teflonschalen (Rotilabo-Dosen, Roth, Karlsruhe, Deutschland) bei 37°C und 5 % CO₂ für zwei Stunden inkubiert, dann geerntet und 10 Minuten bei 150 g und 22°C zentrifugiert. Anschließend wurden die Zellen gezählt und wiederum auf Teflonschalen ausgesät, in Komplettmedium mit GM-CSF und IL-4 (Zelldichte 1 x 10⁶ pro ml) und über Nacht kultiviert. Am nächsten Tag wurden die Zellen zur weiteren Verwendung geerntet.
2. Gefrorene reife (Tag 7) DC wurden wie unter 1 beschrieben aufgetaut, nach einer Ruheperiode von zwei Stunden bei 37°C und 5 % CO₂ auf Teflonschalen wurden die Zellen geerntet (Zentrifugation 10 min bei 150 g und 22°C) zur weiteren Verwendung.
3. Gefrorene reife (Tag 7) DC wurden wie unter 1 beschrieben aufgetaut und nach einer Ruheperiode von zwei Stunden bei 37°C und 5 % CO₂ auf Gewebekulturschalen (Falcon Bacton Dickinson Labware, New Jersey, USA) wurden die Zellen geerntet (Zentrifugation für 10 min bei 150 g und 22°C) zur weiteren Verwendung.
4. Gefrorene reife (Tag 7) DC wurden in einem 56°C Wasserbad aufgetaut, dann in 10 ml eiskalte "Hank's Balanced Salt Solution" (Bio Whitaker) gegeben und sofort für 12 min bei 133 g und 4°C zentrifugiert. Anschließend wurden die Zellen zur weiteren Verwendung geerntet.

### c) Ermittlung der DC Überlebensrate

Die Überlebensrate eingefrorener und wiederaufgetauter DC wurde durch Kultivierung in Komplettmedium ohne Zusatz von GM-CSF und IL-4 über wenigstens 4 Tage untersucht (= "Washout Test") und mit der Überlebensrate von DC verglichen, die frisch hergestellt worden waren aus nicht eingefrorenen oder gefrorenen Aliquots von PBMC wie beschrieben (Thurner et al. (1999) J. Immunol. Methods 223, 1). Die jeweiligen Mengen an lebenden DC wurden durch einen Zellzähler (Cassy Cell Counter and Analyser System, Model TT, Schärfe System, Reutlingen, Deutschland; dieses System benutzt "Pulse area analysis" und erlaubt die Bestimmung von Zellzahlen, Zellgröße und Volumen sowie ob lebende Zellen vorliegen) und als Kontrolle ebenfalls durch Standard-Trypanblaufärbung bestimmt.

Zunächst wurde der Einfluß der DC-Konzentration untersucht, wobei Einfrieren bei 10 x 10⁶ reifen DC/ml die besten Ergebnisse lieferte. Das Ergebnis ist in Figur 1 dargestellt.

Weiterhin wurde der Einfluß der DMSO-Konzentration (5 bis 12,5 % v/v Endkonzentration) untersucht. Eine Veränderung der DMSO-Konzentration hatte keinen signifikanten Effekt auf die Überlebensrate aufgetauter DC.

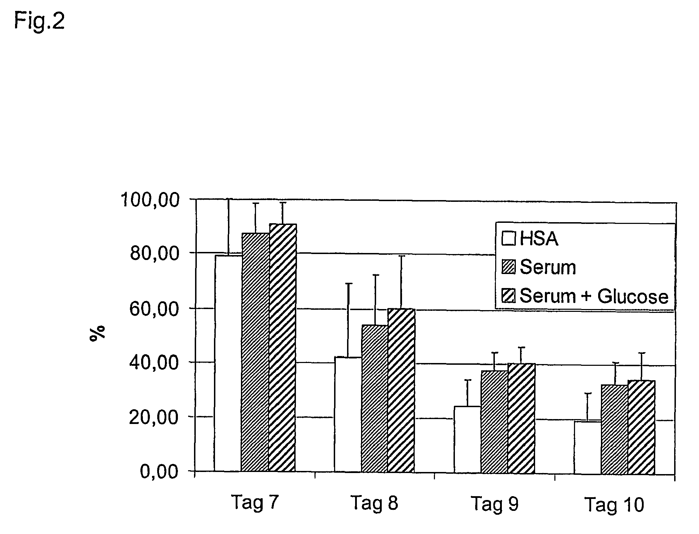

Weiterhin wurde untersucht, ob HSA oder reines autologes Serum bessere Überlebensraten ergibt, sowie ob ein Zusatz von Glukose (Endkonzentrationen v/v von 1, 2, 3, 5, 10 und 15 %) die Überlebensrate verbessern. Das Ergebnis ist in Figur 2 dargestellt. Die Überlebensrate von DC nach mehreren Tagen wurde reproduzierbar erhöht, wenn HSA durch autologes Serum ersetzt wurde. Zusatz von Glukose in einer Endkonzentration von 5 % (v/v) verbesserte weiterhin die Ergebnisse.

Es wurden auch verschiedene kommerziell erhältliche Einfriermedien wie Erythrocyde Freezing Solution^{™} oder Cell Processing Solution (Fresenius) untersucht. Diese lieferten jedoch schlechtere Ergebnisse als die bereits getesteten Einfriermedien.

Es wurden ebenfalls die verschiedenen unter b) genannten Auftaubedingungen überprüft, um den Streß, dem die Zellen nach dem Auftauen ausgesetzt sind, zu minimieren. Keine der vier getesteten Methoden zeigte eine klare Überlegenheit gegenüber den anderen.

Reife (Tag 7) DC wurden hergestellt wie in Beispiel 1 beschrieben und unter folgenden Bedingungen eingefroren:
Abkühlung 1°C pro min in reinem autologem Serum + 10 % DMSO + 5 % Glukose bei einer Zelldichte von 10 x 10⁶/ml. Nach einer mindestens dreistündigen Aufbewahrung in der Gasphase von flüssigem Stickstoff wurden die Zellen aufgetaut. Nach dem Auftauen wurde der Prozentsatz an lebenden DC direkt bestimmt (= D7). Aliquots der DC wurden ausgesäht und die Überlebensraten nach bis zu 4 Tagen in Kultur im Medium ohne Cytokine ("Washout Test") bestimmt wie in Beispiel 1c) dargestellt (n = 20). Das Ergebnis ist in nachstehender Tabelle dargestellt.

### Ausbeute an gefrorenen/aufgetauten DC nach Auftauen (Tag 7) und in "Washout Tests" (Tage 8 bis 11)

| Tage | Ausbeute in % | 95 % Konfidenzinterval |
|---|---|---|
| 7 | 93,3 | 100,0-85,8 |
| 8 | 74,1 | 81,7-66,6 |
| 9 | 63,3 | 70,8-55,7 |
| 10 | 55,8 | 63,4-48,2 |
| 11 | 47,1 | 57,9-36,2 |

### Beispiel 3

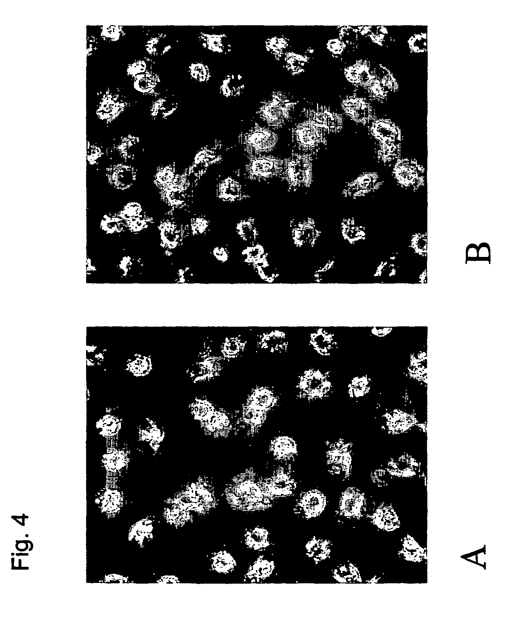

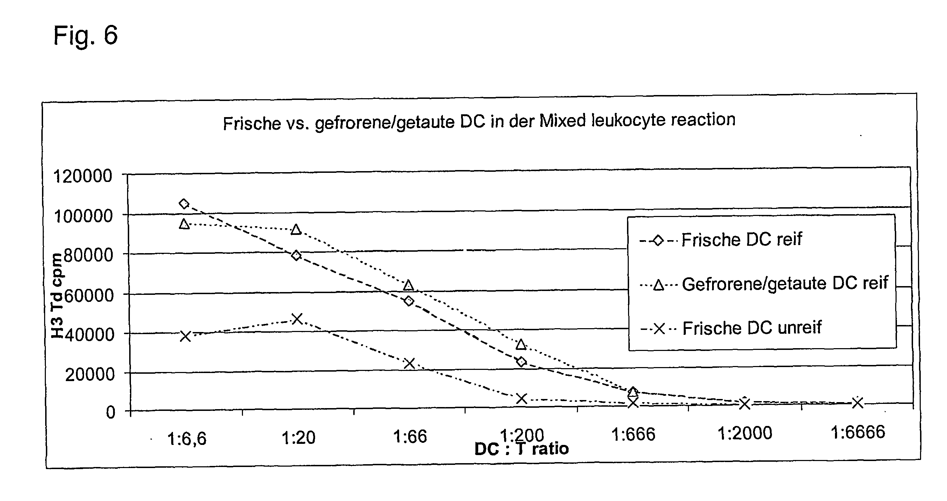

Optimal gereifte und eingefrorene DC sind frisch hergestellten DC gleichwertig im Hinblick auf Überlebensrate und T-Zell-stimulatorische Aktivität.
a) Zunächst wurde untersucht, ob die Überlebensrate eingefrorener und wieder aufgetauter DC vergleichbar ist mit der Überlebensrate frisch hergestellter DC aus demselben Spender. Dazu wurde der "Washout Test" benutzt, wie er in Beispiel 2c) beschrieben ist. Das Ergebnis ist in Figur 3 dargestellt. Es wurde gefunden, dass kein Unterschied besteht zwischen aufgetauten DC und frisch hergestellten DC aus demselben Spender.
   Es wurde ebenfalls die Morphologie und der Phänotyp aufgetauter DC mit frisch hergestellten DC verglichen. Die Morphologie der Zellen wurde unter einem Invert-Phasenmikroskop (Leika DM IRB, Leika Mikroskopie und Systeme GmbH, Wetzlar, Deutschland) untersucht und photographisch festgehalten. Der Phänotyp der Zellpopulationen wurde untersucht durch eine Reihe monoklonaler Antikörper und auf einem FACScan-Gerät untersucht (Becton Dickinson, New Jersey, USA) wie beschrieben (Thurner et al. (1999) J. Immunol. Methods 223, 1). Tote Zellen wurden aufgrund ihrer Lichtstreuungseigenschaften aussortiert. Das Ergebnis ist in den Figuren 4 und 5 dargestellt. Gefrorene und wiederaufgetaute DC behalten ihre charakteristischen morphologischen Eigenschaften und ihren Phänotyp über mehrere Tage hinweg.
b) Sodann wurde untersucht, ob wiederaufgetaute DC auch ihre funktionalen Eigenschaften beibehalten.
   1. Dazu wurde untersucht, ob wiederaufgetaute DC genauso effektiv wie frisch hergestellte, nicht eingefrorene reife DC primäre allogene MLRs induzieren können. Dieser Test wurde durchgeführt wie beschrieben (Thurner et al. (1999), J. Immunol. Methods 223, 1). DC wurden in abgestuften Dosen zu 2 x 10⁵ allogenen T-Zellen pro Weil in 96 Weil-Platten mit flachem Boden gegeben und 4 bis 5 Tage in RPMI 1640 (supplementiert mit Gentamicin, Glutamin und 5 % allogenem hitzeinaktiviertem humanem Serum (Pool Serum)) co-kultiviert, und die Proliferation wurde durch Zugabe von ³H-Thymidin (4 µCi Endkonzentration/ml) für die letzten 12 bis 16 Stunden der Co-Inkubation bestimmt. Das Ergebnis ist in Figur 6 dargestellt. Eingefrorene und wiederaufgetaute DC induzieren primäre allogene MLRs genausogut wie frisch hergestellte reife DC.
   2. Es wurde auch untersucht, wie gut eingefrorene und wiederaufgetaute DC zytotoxische T-Lymphozyten (CTL) induzieren können. Dazu wurde die Induktion von IMP (Influenza Matrix Peptide)-spezifischen CTL durch IMP-gepulste reife DC gemessen. Dieser Ansatz ist spezifisch für reife DC, wenn er in Abwesenheit von T-Zellhilfe und exogenem IL-2 durchgeführt wird. Die Induktion von Influenza Matrix A2.1-Peptid (IMP) oder Melan-A A2.1-Peptid-spezifischen CD8+ T-Zellen wurde durchgeführt durch Stimulation gereinigter CD8+ T-Zellen (isoliert aus PBMC durch magnetische Zellsortierung/MACS mit CD8-Mikrokügelchen nach den Angaben des Herstellers, Miltenyi Biotec, Bergisch Gladbach, Deutschland) oder in anderen Experimenten von nichtadhärenten PBMC-Fraktionen mit DC (hergestellt aus autologem PBMC von HLA-A2.1+-Spendern), die entweder ungepulst oder gepulst waren mit HLA-A2.1-beschränktem IMP (GILGFVFTL [SEQ ID NO:1], 10 µM für 1 Stunde bei 37°C bei 1 x 10⁶ DC/ml Komplettmedium) oder Melan-A-analog Peptid (ELAGIGILTV [SEQ ID NO:2], 10 µm) bei einem DC/T-Verhältnis von 1:10 oder 1:30 für 7 Tage ohne Zugabe von Cytokinen. CTL wurden durch einen Standard-Lyse-Assay quantifiziert (Bhardwaj et al. (1994) J. Clin. Invest. 94, 797) oder durch Tetramerfärbung bei 37°C (Whelan et al. (1999) J. Immunol. 163, 4342). Zielzellen für den Standard-4 Stunden-⁵¹Cr-Release Assay, der bei verschiedenen Effektor/Zielzellverhältnissen durchgeführt wurde, waren IMP-gepulste (10 µg/ml für 1 Stunde bei 37°C) T2A1-Zellen, nichtgepulste T2A1-Zellen und K562-Zielzellen (alle ⁵¹Cr-markiert). Alle Versuche wurden mit einem 80-fachen Überschuß an K562-Zellen durchgeführt, um die natürliche Killerzellaktivität zu blockieren. Die spezifische Lyse in % wurde berechnet durch die Formel (spezifische Freisetzung - spontane Freisetzung)/(maximale Freisetzung - spontane Frei-setzung) x 100. Lösliche IMP- und Melan-A/HLA A2.1-Tetramere wurden hergestellt, und die Bindung an T-Zellen wurde analysiert durch Durchflußzytometrie bei 37°C wie beschrieben (Whelan et al., 1999). 1 µl Tetramer (0,5 bis 1 mg/ml) wurde zu 2 x 10⁶ Zellen in etwa 60 µl (zurückbleibendes Volumen im Gefäß nach Zentrifugation und Abgießen des Überstands) Medium, bestehend aus RPMI 1640, mit Gentamicin, Glutamin und 5 % allogenem hitzeinaktivierten humanem Serum (Pool Serum) supplementiert, für 15 min bei 37°C gegeben. Anschließend wurden die Zellen ohne Waschen abgekühlt und für 15 min mit einem dreifach gefärbten monoklonalen Antikörper gegen humanes CD8 (Caltag Laboratories, Burlingame, Kalifornien) auf Eis inkubiert. Nach drei Waschschritten
   wurden die Zellen auf einem FACScan-Gerät analysiert (Becton Dickinson).
   Das Ergebnis ist in den Figuren 7 und 8 dargestellt.
   Einfrieren und Auftauen von DC ändert nicht die Fähigkeit reifer DC eine starke IMP-spezifische CLT-Antwort zu induzieren. Einfrieren und Auftauen ändert ebenfalls nicht die Fähigkeit reifer DC, starke IMP-spezifische CD8+-T-Zellantworten zu induzieren, wie durch die HLA-A2.1/Peptid Tetrameranalyse gezeigt.

Diese Versuche zeigen, dass nach Einfrieren und Auftauen lebende DC erhalten wurden, die vollkommen gleichwertig zu frisch hergestellten DC sind.

### Beispiel 4

Um eine erhöhte Überlebensrate der eingefrorenen und wieder aufgetauten DC zu erreichen, wurden die folgenden verschiedenen anti-apoptotischen Stimuli in verschiedenen Konzentrationen und zu unterschiedlichen Zeitpunkten zu den DC gegeben:
1. Rekombinantes murines oder menschliches trimeres TRANCE (Wong et al. (1997) J. Exp. Med. 186, 2075) zu 100, 200, 500 ng/ml;
2. RANKL (Anderson et al. (1997) Nature 390, 175) zu 10 ng/ml, 50 ng/ml, 100 ng/ml und 1 µg/ml;
3. Trimeres, lösliches CD40L (Morris et al. (1999) J. Biol. Chem. 274, 418) zu 50, 100 und 500 ng/ml.

Die DC wurden den verschiedenen anti-apoptotischen Stimuli bei 37°C über Nacht für die letzten 12-16 h der Kultur vor dem Einfrieren, für 4 h vor dem Einfrieren (Zelldichte 1x10⁶ in Komplettmedium mit GM-CSF und IL-4) und ebenfalls für 4 h nach dem Auftauen (Zelldichte 1x10⁶ in Komplettmedium mit GM-CSF und IL-4) ausgesetzt.

Eine kurzzeitige Exposition aufgetauter DC mit den anti-apoptotischen Stimuli war genauso wirksam wie die DC-Behandlung vor dem Einfrieren und ergab eine erhöhte Überlebensrate, die allerdings oft erst nach 3 Tagen im "Washout test" sichtbar wurde. CD40L (Figur 9) und TRANCE/RANKL (Ergebnisse nicht gezeigt) ergaben ähnliche Resultate. Die Ergebnisse zeigen, dass Zugabe von CD40L oder TRANCE/RANKL die Überlebensrate von DC über Tag 3 hinaus verbessert.

### Beispiel 5

Um zu untersuchen, ob DC vor dem Einfrieren erfolgreich mit Antigen beladen werden können, wurden DC mit Tetanus Toxoid (TT) (als Beispiel für ein Protein-Antigen) oder mit IMP (als Modellpeptid) beladen. Zum Pulsen mit TT wurden DC aus frischen oder eingefrorenen Aliquots von PBMC aus Leukaphereseprodukten hergestellt (s. o.). TT wurde den unreifen Zellen an Tag 5 zu 10 µg/ml zugesetzt. Reife DC wurden an Tag 7 geerntet und entweder uneingefroren oder nach Einfrieren und Auftauen (nach 4 h) auf ihre Fähigkeit hin untersucht, TT-spezifische proliferative Antworten in PBMC zu induzieren. Dazu wurden abgestufte Dosen an ungepulsten und TT-gepulsten DC zu PBMC (10x10⁴/well) gegeben und an Tag 5 mit ³H Thymidin gepulst wie beschrieben (Thurner et al. (1999) J. Immunol. Methods 223, 1). Zur Beladung mit IMP wurden DC mit 10 µM Peptid (für 1 h, 37°C, 1x10⁶ DC/ml Komplettmedium) gepulst entweder vor dem Einfrieren oder nach dem Auftauen. Die Fähigkeit, IMP erfolgreich zu präsentieren, wurde getestet wie obenstehend beschrieben.

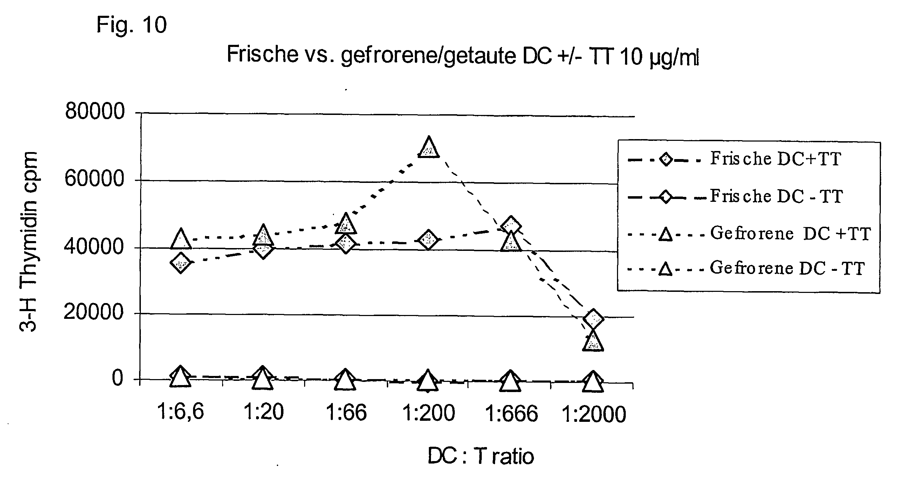

Eingefrorene, TT-gepulste DC waren genauso stimulatorisch wie frisch hergestellte, TT-gepulste DC (Figur 10). Sowohl vor dem Einfrieren als auch nach dem Auftauen IMP- bzw. Melan A-beladene DC stimulierten gleich gut IMP- bzw. Melan A-spezifische CTL (Figuren 8 und 11). Diese Ergebnisse zeigen, dass es möglich ist, eingefrorene Aliquots reifer DC herzustellen, die bereits mit Antigen beladen sind und nach dem Auftauen unmittelbar verwendet werden können.

### Beispiel 6

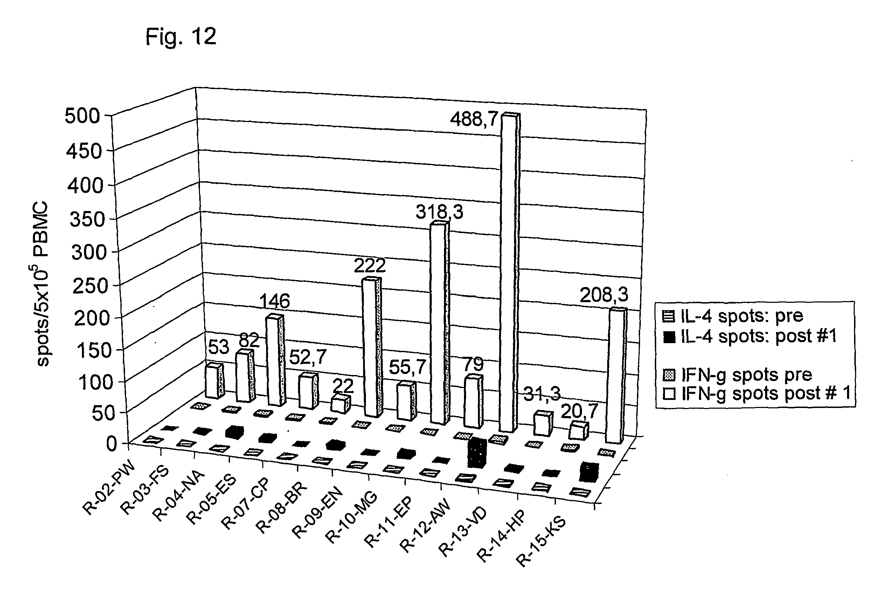

Es wurde bei Stadium IV Melanom-Patienten eine Vakzination mit Peptid- und Protein-beladenen DC, welche nach den hier dargestellten Verfahren hergestellt und mit Antigen beladen wurden, durchgeführt. Figur 12 zeigt die Induktion einer Helferzell Typ 1 Antwort gegen KLH in allen vakzinierten Patienten. Die Patienten erhielten jeweils nur eine einzige subkutane Gabe von 4 Mio. reifen DC, welche aus Leukopheresaten unter Verwendung von GM-CSF und Interleukin-4 und der Reifungszusammensetzung wie in der Anmeldung geschildert, hergestellt wurden (z. B. Figur 1 und 3). Es wurde hierbei das Kontrollantigen KLH in einer Konzentration von 10 µg/ml gleichzeitig mit der Reifungszusammensetzung zugegeben und die DC dann portioniert eingefroren. Vor der Vakzination und 14 Tage nach der einmaligen Verabreichung von 4 Mio. DC wurde Blut entnommen und mittels Standard-Elispot-Assay (Zugabe von 10 µg/ml KLH zu 500.000 PBMC und Messung der Anzahl der Interferon-γ oder IL-4 produzierenden Zellen; der Hintergrund ohne Zugabe von KLH ist nahezu 0). Es zeigt sich, dass vor der Vakzination weder Interferon-γ noch Interleukin-4 nach KLH-Präsentation produziert wird, wogegen 14 Tage nach der einmaligen Impfung mit den KLH beladenen DC in allen Patienten Interferon-γ, aber nicht oder nur minimal Interleukin-4 produzierende T-Zellen induziert wurden (in Kontrollexperimenten wurde durch Entfernen der CD4-Zellen aus den PBMC gezeigt, dass es sich bei den reaktiven Zellen um CD4-positive Helfer-T-Zellen handelt).

### Beispiel 7

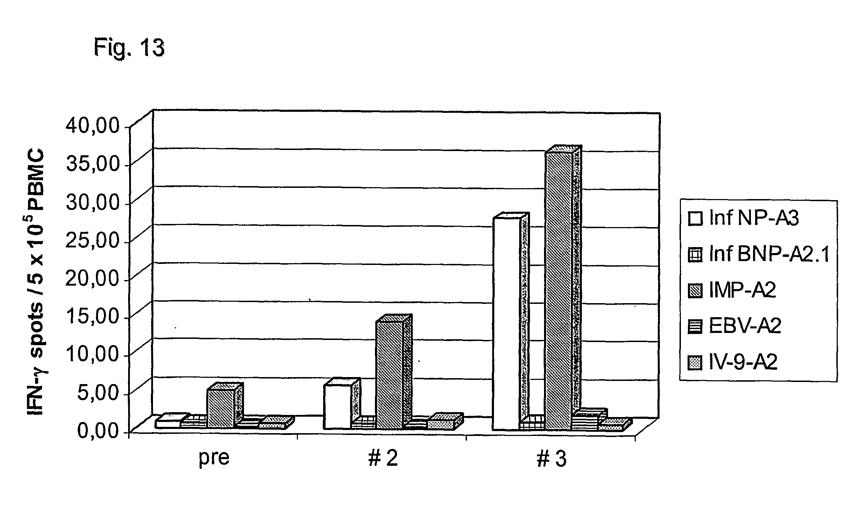

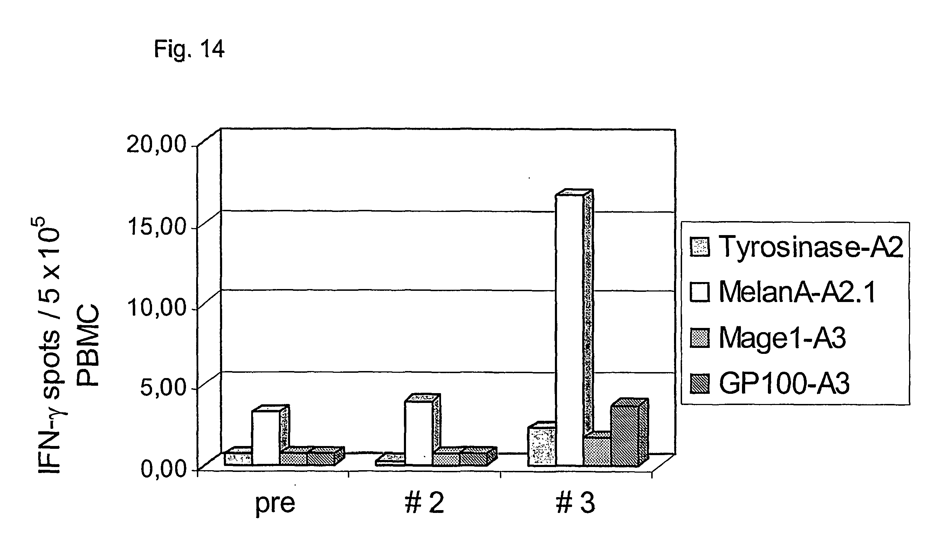

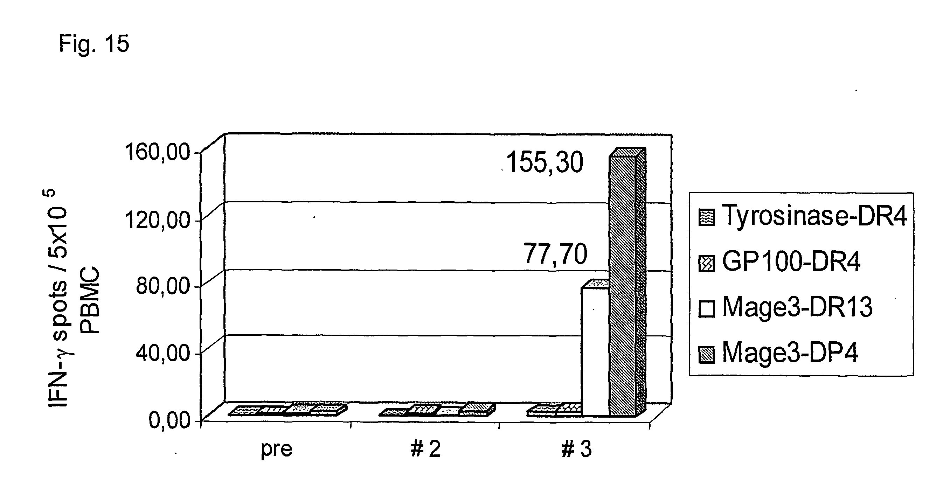

Ein Patient aus der Studie von Beispiel 6 erhielt mehrere Vakzinationen mit DC, wobei jeweils 4 Mio. wieder aufgetaute DC mit den in Figur 13, 14 bzw. 15 angeführten Peptiden beladen (d. h. pro 4 Mio. DC jeweils nur 1 Peptid) und subkutan injiziert wurden. Jeweils vor der ersten Impfung und 14 Tage nach der jeweils vorangehenden Impfung und unmittelbar vor der nächsten Impfung wurde Blut entnommen und ein Standard-Elispot-Assay durchgeführt wie in Thurner et al. (1999), J. Exp. Med. 190, 1669-1678 unter Material und Methoden beschrieben. Die für die Vakzination verwendeten DC wurden wie in der Legende zu Figur 8 und 11/in Beispiel 5 geschildert hergestellt und erst nach dem Auftauen mit den entsprechenden Peptiden beladen. Wie aus den Figuren 13 bis 15 ersehen werden kann, wird gegenüber mehreren Klasse-I- (Figuren 13 und 14) und Klasse-II-Peptiden (Figur 15) Immunität induziert. Die Charakteristika der verwendeten Peptide sind Tabelle I zu entnehmen. Anzumerken ist, dass der HLA-Typ des speziellen Patienten HLA-A 2.1+ und HLA-A3+ ist sowie HLA-DR 13+, HLA-DP4+ (aber DR4-). Figur 13 zeigt die Induktion von Immunität gegen die Influenzapeptide NP-A3 und IMP-A2 nach einer einzigen Impfung (Nr. 2 bedeutet Blutentnahme und Elispot-Messung kurz vor Verabreichung der Vakzination Nr. 2) und zeigt einen Anstieg nach einer weiteren Impfung, aber keine Änderung bei EBV-A2 und IV-9-A2 Peptiden, welche zur Impfung nicht verwendet wurden. Figur 14 zeigt eine Induktion der Immunität gegen 4 Klasse-I-restringierte Tumorpeptide, deutlich und zweifelsfrei gegen das Peptid Melan A-A2.1. Figur 15 zeigt eine Induktion von Immunität gegen zwei Klasse II-restringierte Tumorpeptide (Mage 3-DR13 und -DP4), nicht aber gegen Tyrosinase-DR4 und GP 100 DR4 (dies stellt eine Negativkontrolle dar, da der Patient DR4 negativ war und die DC somit nicht mit diesen Peptiden beladen werden konnten).

### Beispiel 8

### Kryokonservierung von Dendritischen Zellen nach der Antigenbeladung mit Tumorzellpräparationen (Tumor-Zell-Lysate, Nekrotische oder apoptotische Tumorzellen)

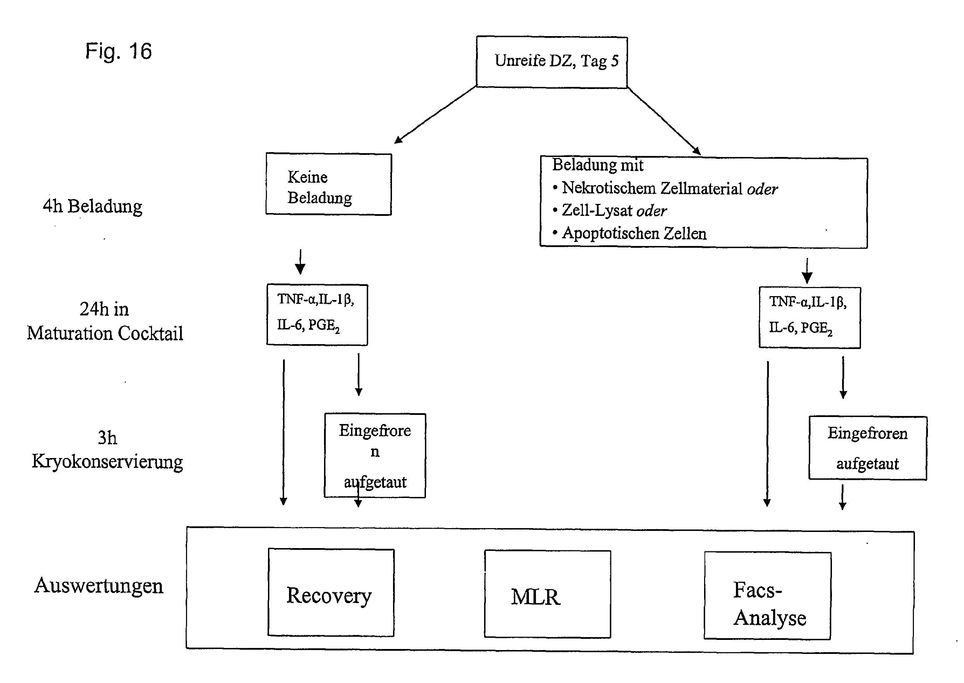

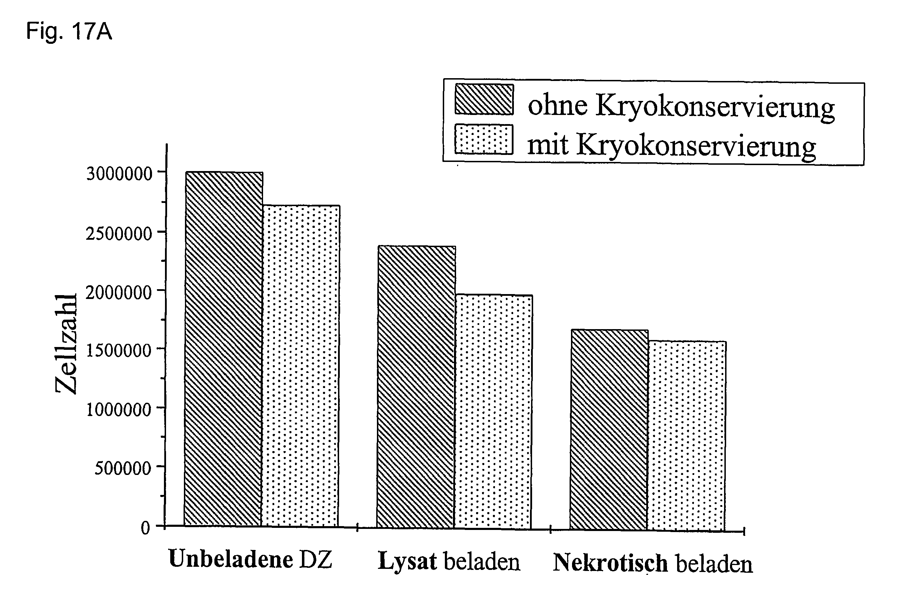

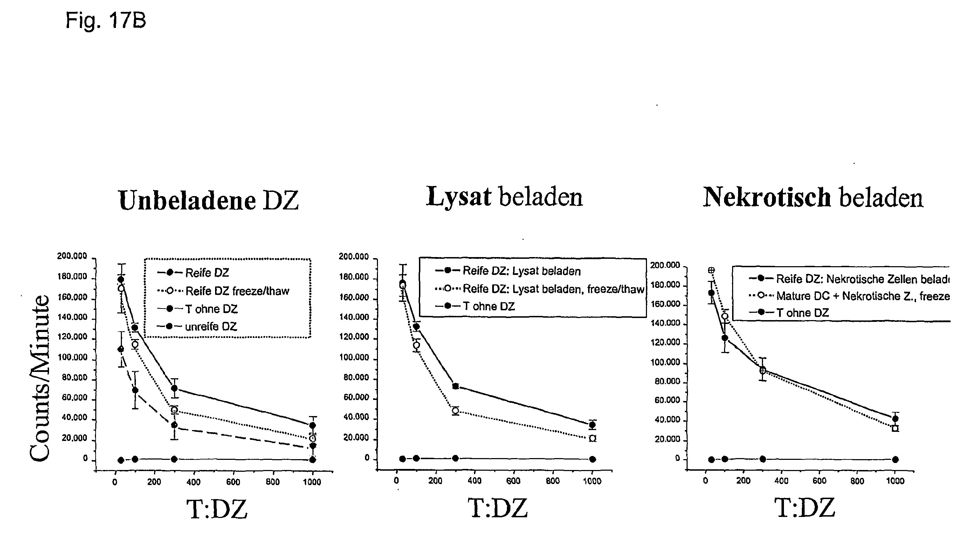

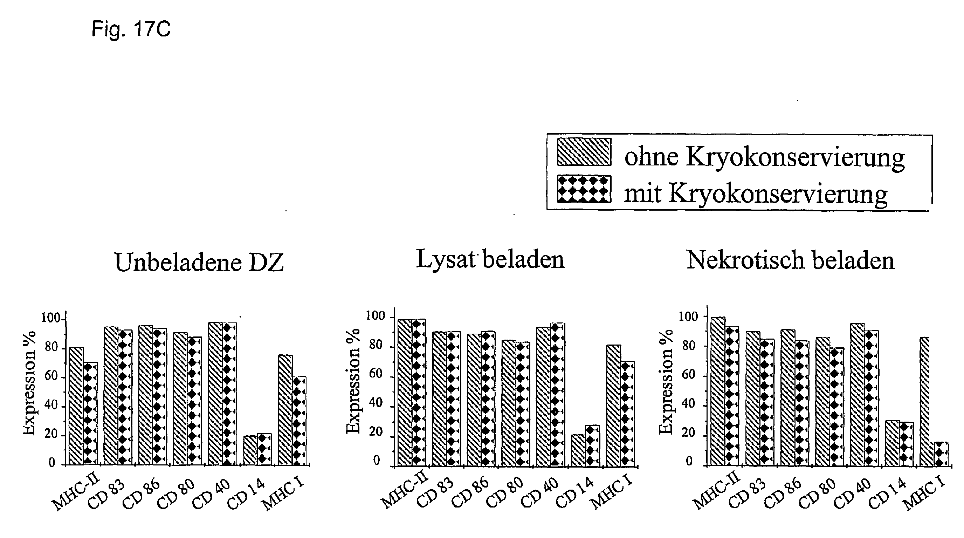

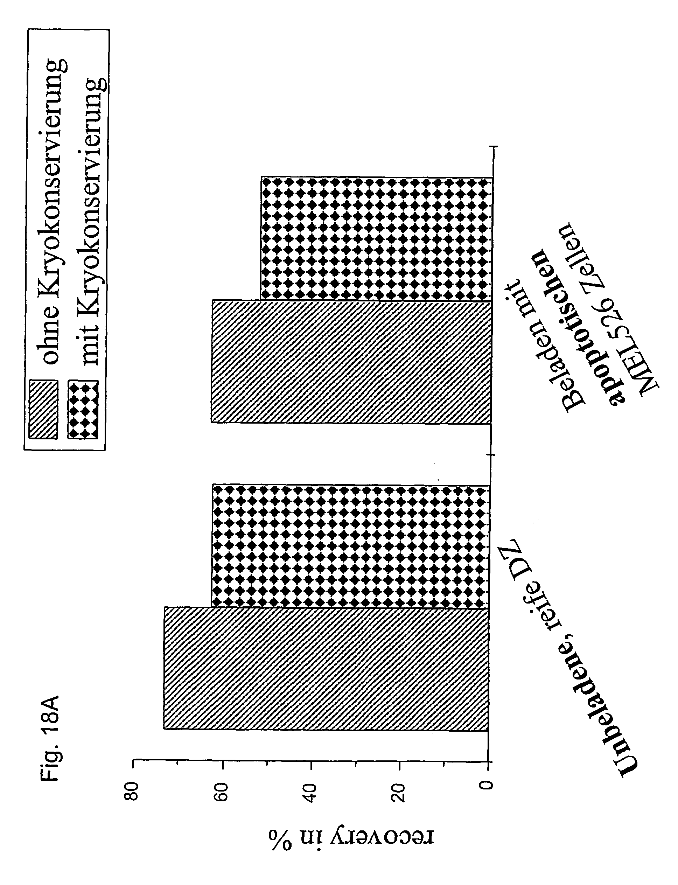

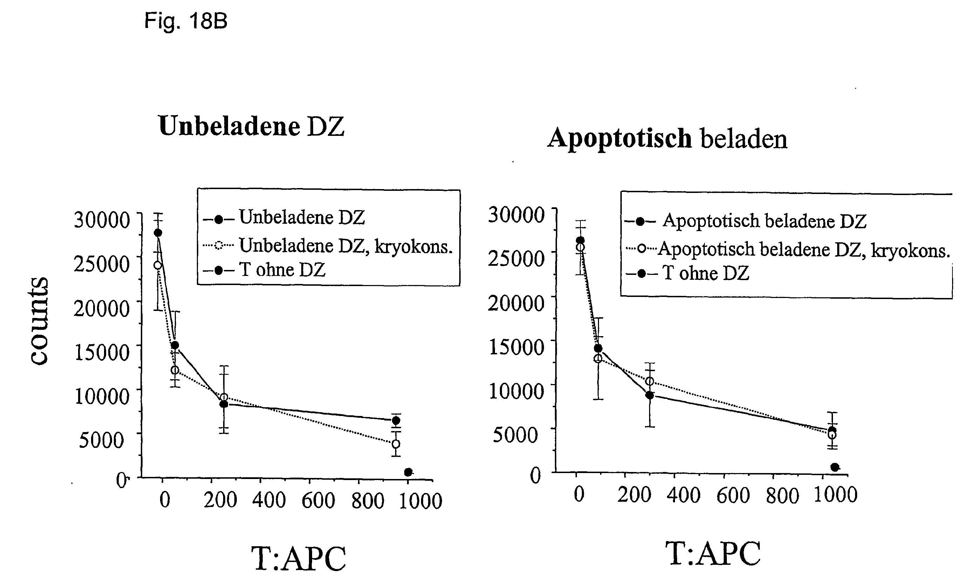

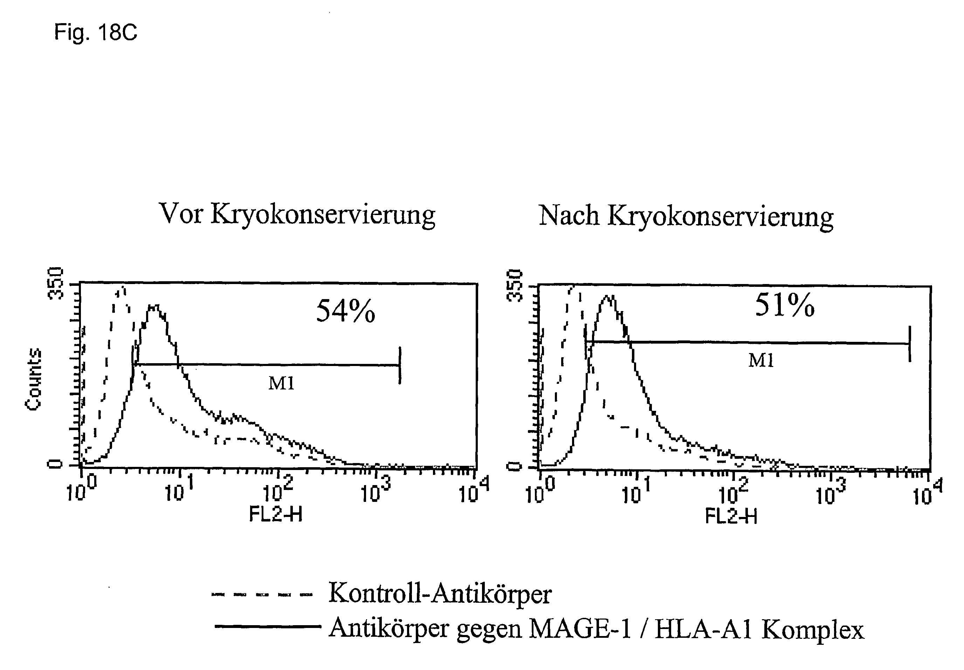

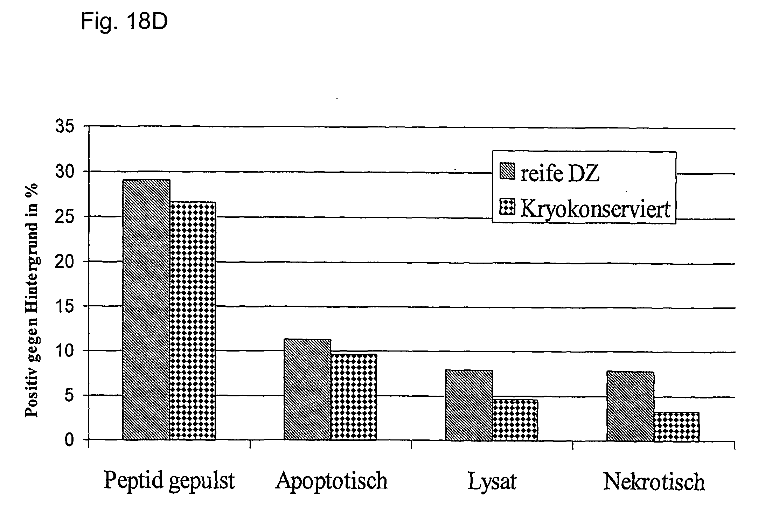

Dendritische Zellen (DC) können in großer Menge aus Leukapheresen generiert werden. Es wurde eine Methode zur Kryokonservierung reifer DC entwickelt, die erlaubt die portionierte Verwendung dieser Zellen zur Vakzination erlaubt. Bisher wurden die reifen DC vor der Anwendung mit Peptiden beladen, die den immunodominanten Sequenzen von Tumor assoziierten Antigenen (TAA) entsprechen. In den hier beschriebenen Versuchen (siehe Figur 16) wurden überprüft, ob unreife DC, die mit verschiedenen Tumorzellpräparationen beladen und anschließend gereift wurden, ebenfalls kryokonserviert werden können.
Tumorzellpräparation: Zur Herstellung der Tumorzellpräparationen wurde die Mel526 Melanomzellinie verwendet. Mel526 Zellen wurden in RPMI gewaschen und durch repetetive Erhitzung auf 57°C und anschließende Kühlung im Flüssigstickstoff behandelt. Anschließend wurde das Zellmaterial mit Hilfe eines Ultraschallgerätes zertrümmert. Da durch die Erwärmung/Einfrierzyklen Nekrose induziert wird, wird diese Art der Tumorzellpräparation, die sämtliche Zellbestandteile enthält, im Folgenden nekrotisches Zellmaterial genannt. Um Lysat zu gewinnen haben wir an diese Schritte eine Ultrazentrifugation angeschlossen um Zellbestandteile zu entfernen und eine Aufreinigung der Proteine im Centricon-Zentrifugationstube durchgeführt. Wir haben nach den Ergebnissen der Bradford Analyse die Proteinfraktion mit der höheren Aktivität, nämlich die ≥ 10 KDa. verwendet. Apoptotische Tumorzellen wurden mit einem Breitspektrum UVB Bestrahlungsgerät induziert und mit einem Annexin V Test verifiziert.
Generierung der DC: DC wurden entsprechend der in der Experimentellen Immuntherapie verwendeten Technik aus Leukapheresen generiert. PBMC wurden in Nunc Cell Factories ausgeplated und mit RPMI (1 % autologes, hitzeinaktiviertes Plasma) substituiert mit 1000 IU/ml GM-CSF und 500 IU/ml IL-4 kultiviert. Am Tag 5 wurden die dann unreifen DC verwendet und für 4 Stunden mit den geschilderten Tumorzellpräparationen beladen in einer Konzentration von 1 : 1.
Beladung: Die Beladung erfogte in einem 5 ml Polypropylene Reaktionsgefäß bei 37°C und 5 % CO₂. Nach der Beladung wurden die DC in 12 ml Tissue culture plates ausgeplated und mit einem Maturationscocktail bestehend aus TNF-α, IL-1β, IL-6 und PGE₂ für 24 h kultiviert.
Einfrieren/Auftauen: jeweils die Hälfte der jeweils beladenen DC wurde entsprechend der beschriebenen Methode (Feuerstein B. et al., J. Immunol. Methods 245: 15-29 (2000)) in 1,8 ml Nunc freeze vials bei -80 °C für 3 Stunden kryokonserviert. Danach wurden die Zellen wiederum entsprechend der beschriebenen Methode aufgetaut und für eine Stunde im RPMI Medium bei 37°C und 5 % CO₂ kultiviert. Anschließend wurde diese Fraktion der DC wie die nicht eingefrorene Fraktion analysiert und für weitere Versuche verwendet.
Der beschriebene Versuchsaufbau ist als Flussdiagramm in Figur 16 dargestellt.
Zellzahl und Viabilität: Die Gesamtzellzahl und die Viabilität der DC nach der Durchführung der geschilderten Beladung und der Kryokonservierung wurde mit Trypan Blau im Mikroskop bestimmt. Anfänglich werden 5 x 10⁵ DC verwendet. Wir fanden vergleichbare Zellzahlen und keinen Unterschied in der Viabilität der beladenen und kryokonservierten DC im Vergleich zu den nicht kryokonservierten Zellen (Figur 17A, 18A). Eine Abnahme der Zellzahl durch die Beladung, *nicht* aber durch die Kryokonservierung, insbesondere mit nekrotische Zellen war zu beobachten.
Funktionalität: Zur Testung der funktionellen Kapazität wurde ein Mixed-Leukocyte-Reaction-Test (MLR) durchgeführt. Die beladenen DC wurden in einem allogenen MLR eingesetzt (4 Tage Inkubation mit allogenen Leukozyten) und dann mit radioaktivem Thymidin (³H-Thymidin) für 13 Stunden gepulst. Es ergaben sich vergleichbare allostimulatorische Potenzen für kryokonservierte und nicht kryokonservierte beladene DC (Figur 17B, 18B).
Phänotyp: Zur Beurteilung der Expression der für die Antigenpräsentation und den Funktionszustand der DC relevanten Oberflächenmoleküle wurde eine FACS-Analyse mit den entsprechenden Antikörpern durchgeführt. Es wurden eine vergleichbares Oberflächenexpressionsmuster sowohl für die verschiedenen Beladungsmethoden als auch nach der Kryokonservierung gefunden (Figur 17C).
Direkter Antigennachweis: Zum direkten Nachweis von tumoralem Antigen wurde ein Antikörper eingesetzt, der MAGE-1 im HLA-A1 Kontext d. h. den Komplex aus Mage-1 Peptid und dem HLA-A1 Molekül erkennt. Die verschieden beladenen DC wurden mit diesem Antikörper gefärbt und im FACS analysiert. Dabei zeigte sich bei Verwendung von peptidbeladenen (20µg MAGE-1 Peptid für 3 Stunden/ml) DC, dass nach der Kryokonservierung ein vergleichbarer Prozentsatz von MAGE-1/A1 Antigen nachweisbar war wie ohne Kryokonservierung (Figur 18C). Aus diesem Versuch lässt sich schließen, dass die Kryokonservierung nicht zu einem Antigenverlust führt.
In einem weiteren Versuch wurden der MAGE-1/A1 Antikörper angewendet, um die Effektivität der verschiedenen Beladungsmethoden zu bestimmen. Mit den Tumorzellpräparationen (die verwendete Melanomzell-Linie exprimiert das Mage-1 Antigen) konnte eine signifikante Beladung erreicht werden, die etwa 20 % der Antigendichte des Peptidpulsens erreichte (Figur 18D). Dabei war die Expression des MAGE-1/HLA-A1 Komplexes vor und nach Kryokonservierung in vergleichbarer Quantität nachweisbar (errechnet als Positivität gegen den Hintergrund unbeladener DC).

Schlussfolgerung: Es konnte also gezeigt werden, dass das Verfahren der vorliegenden Erfindung nicht nur eine effektive Kryokonservierung unbeladener, mit Peptid oder Protein beladenener DC erlaubt (wie in Beispiel 1-7 gezeigt), sondern auch eine ebenso effektive von DC, welche mit (Tumor-) Zellpräparationen (simplen nekrotischen Tumorzellen, aus Tumorzellen hergestellten Lysaten oder apoptotischen Tumorzellen) beladen wurden. Effektiv bedeutet, dass durch das Einfrieren i) der Zellverlust nach dem Auftauen ≤ 25 % im Vergleich zu nicht eingefrorenen DC beträgt, ii) die aufgetauten DC eine vergleichbare T Zell-Stimulatorische Kapazität aufweisen wie die nicht eingefrorenen DC (getestet in der allogenen MLR) und iii) die Oberflächen-Expression von Antigenen bzw. Liganden für T Zell-Rezeptoren (d. h. spezifische MHC-Peptidkomplexe) nach dem Einfrier- und Auftauvorgang erhalte bleibt (modellhaft gezeigt durch direkten Nachweis eines bestimmten Peptid/MHC-Komplexes, nämlich des MAGE-1/HLA-A1-Komplexes, mittels eines diesen Komplex spezifisch erkennenden monoklonalen Antikörpers).

### Beispiel 9

### Kryokonservierung von Dendritischen Zellen nach der Antigenbeladung mittels adenoviraler Transfektion

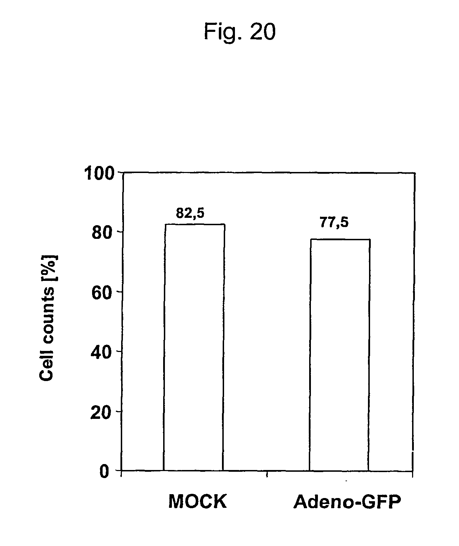

Mit Adenoviren transfizierte Dendritischen Zellen können mittels dem erfindungsgemäßen Verfahren so eingefroren werden, dass die Eigenschaften der Dendritischen Zellen im Vergleich zu nicht transfizierten Dendritischen Zellen vergleichbar sind. Hierzu wurden reife DC bei einer Multiplizität der Infektion (MOI) von 500 für 2 Stunden mit einem Adenovirusvektor (AD5) infiziert, der eine für das "Green Fluorescent Protein" (GFP) kodierende cDNA enthält. Nach zweimaligem Waschen wurden die Zellen bei einer Konzentration von 10 x 10⁶ DC/ml im HSA und 10 % DMSO in 5 % Glukose (Endkonzentration) eingefroren und für 4 Stunden aufbewahrt. Nach Auftauen wurde die Lebensfähigkeit der Zellen durch Trypanblau-Exklusion bestimmt. Die Wiedergewinnungsrate von lebensfähigen Zellen ist in Figur 20 als Prozentsatz der eingefrorenen DC angegeben.

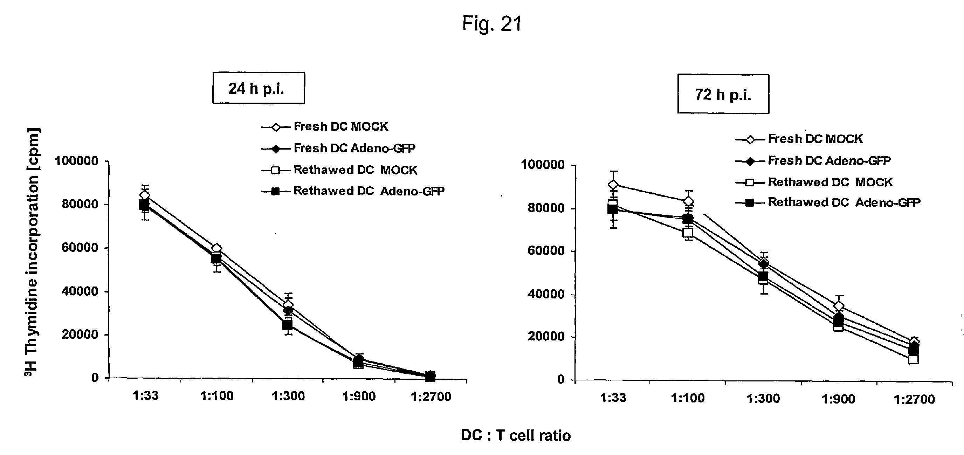

Weiterhin konnte gezeigt werden, dass die allostimulatorische Aktivität von Adenovirus-infizierten DC durch Kryokonservierung nicht verändert wird. Hierzu wurden reife DC mit Adeno-GFP bei einem MOI von 500 infiziert und kryokonserviert, wie vorstehend beschrieben. Nach Wiederauftauen und einer Kulturperiode von 24 oder 72 Stunden wurden die dendritischen Zellen mit allogenischen CD4+-T-Zellen (2 x 10⁵ pro Napf) unter den in Fig. 21 angegebenen Verhältnissen co-kultiviert. Nach 4 Tagen wurden die Zellen mit [³H]-Tymidin für 16 Stunden gepulst und der Einbau der Radioaktivität wurde bestimmt. In Fig. 21 sind die Durchschnittswerte von drei Zählungen mit der entsprechenden Standardabweichungen angegeben. Die Werte von T-Zellen allein oder DC allein waren immer weniger als 1000 pro min.

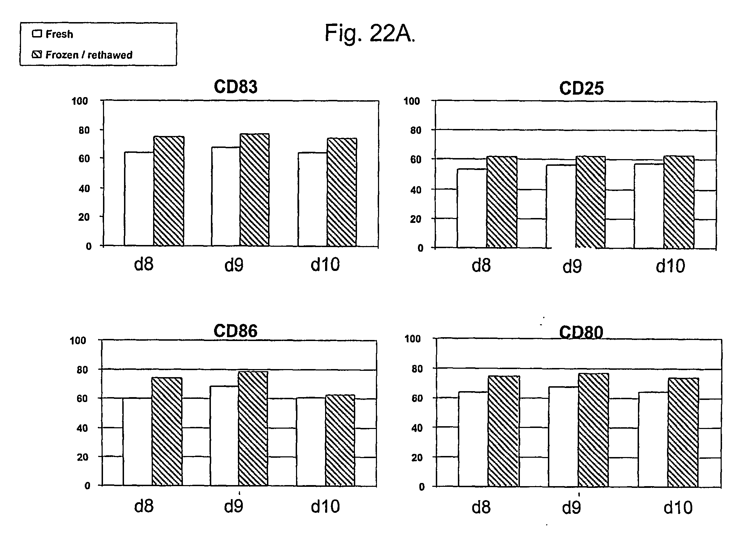

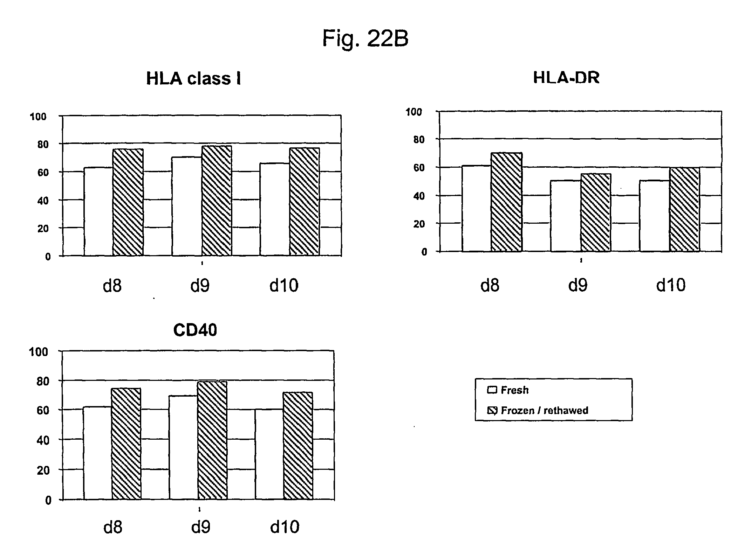

Weiterhin wurden reife DC mit Adeno-GFP bei einem MOI von 500, wie vorstehend beschrieben, kryokonserviert. Nach Wiederauftauen und der angegebenen Kulturzeit wurden die Zellen unter Verwendung von für CD83, CD25, CD86, CD80 spezifischen Antikörpern, gefolgt von PE-konjugiertem Ziegen/Maus-IG-(Fab')₂-Fragmenten gegengefärbt. Die Ergebnisse sind in Fig. 22A gezeigt. Bei einem vergleichbaren Versuch unter Verwendung von für HLA Klasse 1, HLA-DR und CD40 spezifischen Antikörpern, wurden die in Fig. 22B gezeigten Ergebnisse erhalten.

### Beispiel 10

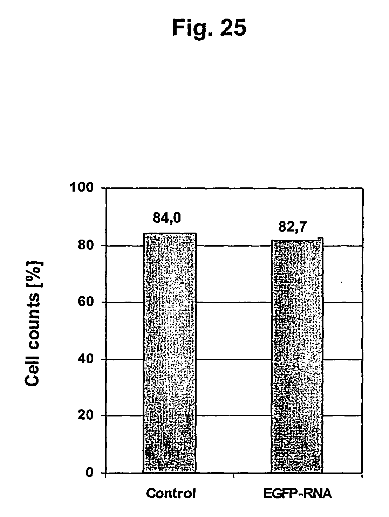

Kryokonservierung von Dendritischen Zellen nach der Antigenbeladung mittels RNA-Transfektion. Mit RNA transfizierte Dendritischen Zellen können mittels des erfindungsgemäßen Verfahrens so eingefroren werden, dass die Eigenschaften der Dendritischen Zellen im Vergleich zu nicht transfizierten Dendritischen Zellen vergleichbar sind. Die DC können in unreifem Stadium mit RNA transfiziert - dann gereift - und als reife DC dann eingefroren werden (nicht gezeigt). Bevorzugt werden bereits reife DC mit RNA transfiziert und kryokonserviert. Die Ergebnisse sind in Figuren 25 bis 27 zusammengefasst.

Hierzu wurden reife dendritische Zellen zweimal mit RPMI gewaschen und einmal in einer Waschlösung des Optimix-Kits (EQUIBIO, Maidstone Kent, UK). DC wurden auf eine Endkonzentration von 40 x 10⁶ DC/ml in Optimix-Medium gebracht. Es wurden dann 0,1 ml der Zellsuspension mit 40 µg in-vitro-transkribierter EGFP-RNA in einem 1,5 ml Reaktionsgefäß vermischt. Nach Inkubierung bei Raumtemperatur für maximal 3 min wurde die Zellsuspension in eine 0,4 cm Spalt-Elektroporationsküvette transferiert und bei einer Spannung von 260 V und einer Kapazität von 150 µF mit dem Gene-Pulser II (Biorad, München, Deutschland) gepulst. Kontroll-DC wurden ohne die Zugabe von RNA gepulst. Die Zellen wurden bei einer Konzentration von 10 x 10⁶ DC/ml in HSA (mit 10 % DMSO und 5 % Glukose (Endkonzentration)) eingefroren und 4 Stunden gelagert. Nach Auftauen wurde die Lebensfähigkeit der Zellen durch Trypanblau-Exklusion bestimmt. Die Wiedergewinnung von lebensfähigen Zellen ist in Figur 25 als Prozentsatz der eingefrorenen DCs angegeben.

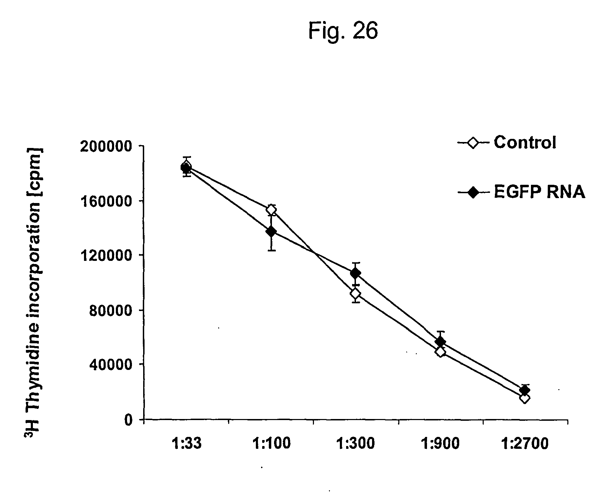

Reife DC wurden mit EGFP-RNA elektroporiert und kryokonserviert, wie vorstehend beschrieben. Nach Wiederauftauen und einer Kulturperiode von 48 Stunden wurden die dendritischen Zellen mit allogenen CD4+-T-Zellen (2 x 10⁵/Napf) bei den in Fig. 26 angegebenen Verhältnissen co-kultiviert. Nach 4 Tagen wurden die Zellen mit [³H]-Thymidin für 16 Stunden gepulst und der Einbau der Radioaktivität wurde bestimmt. In Fig. 26 sind die Mittelwerte bei dreifachen Messung (mit Standardabweichung) gezeigt. Die Zahlen von T-Zellen allein oder DC alleine waren weniger als 1000 pro min.

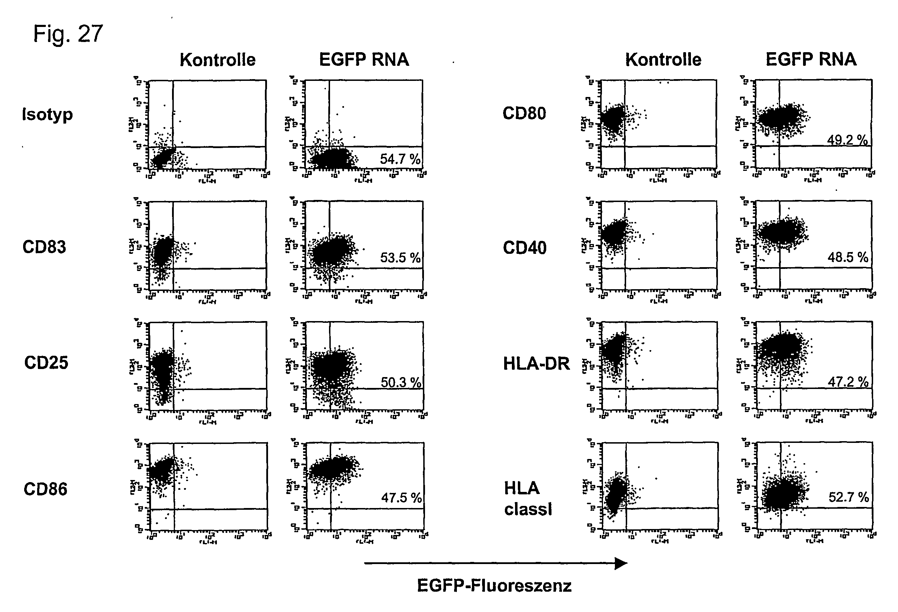

Kryokonservierung von RNA-elektroporierten DC ändert nicht den phenotypischen DC-Marker. Hierzu wurden reife DC elektroporiert, mit oder ohne EGFP-RNA und kryokonserviert, wie vorstehend beschrieben. Nach, Wiederauftauen und einer Kultivierungsperiode von 48 Stunden wurden die DC gegengefärbt mit den in Fig. 27 angegebenen Maus-monoklonalen Antikörpern und PE-konjugierten Antimaus-Ig-(Fab')₂-Fragmenten gefolgt durch FACS-Analyse. Die Zahlen in Figur 27 betreffen im rechten unteren Teil des Quadrats die EGFP-positiven DC, im oberen rechten Teil die EGFP/DC-Marker-doppelpositiven DC.

## Patentansprüche

1. Verfahren zur Herstellung, kryokonservierter, reifer dendritischer Zellen, die nach Auftauen sofort verfügbar sind, umfassend
a) Bereitstellen von unreifen dendritische Zellen (DC);
b) Kultivieren der unreifen DC in einem Kulturmedium, das einen Reifungscocktail mit einem oder mehreren Reifungsstimuli enthält, unter Erhalt von reifen DC;
c) Einfrieren der reifen DC in einem Einfriermedium, das autologes Serum oder autologes Plasma, 5 bis 25% (v/v) DMSO und 2 bis 30% (w/v) einer oder mehrerer Polyolverbindungen enthält.

2. Verfahren nach Anspruch 1, wobei die DC vor dem Einfrieren oder nach Wiederauftauen der eingefrorenen DC mit einem Antigen oder einem Antigen-Antikörperkomplex beladen werden.

3. Verfahren nach Anspruch 1 oder 2, wobei
(i) die unreifen DC aus CD14-positiven, mononuklearen Zellen (Monozyten) oder aus CD34-positiven Zellen hergestellt werden; oder
(ii) die unreifen DC direkt aus dem Blut isoliert werden; oder
(iii) die unreifen DC oder deren Vorläuferzellen aus Leukapheresaten gewonnen werden; oder
(iv) die unreifen DC oder deren Vorläuferzellen aus Frischblut oder Knochenmark gewonnen werden.

4. Verfahren nach einem oder mehreren der Ansprüche 1 bis 3, wobei die Reifungsstimuli ausgewählt sind aus IL-1 wie IL-1β, IL-6, TNF-α, Prostaglandine wie PGE₂, IFN-α, Lipopolysacchariden und anderen bakteriellen Zellprodukten wie MPL (Monophosphoryllipid A) und Lipoteichonsäure, Phosphorylcholin, Calciumionophore, Phorbolester wie PMA, Heat-Schock-Proteine, Nukleotide wie ATP, Lipopeptide, künstliche Liganden für Toll-ähnliche Rezeptoren, doppelsträngige RNA wie Poly-I:C, immunstimulatorische DNA-Sequenzen und CD40-Ligand, insbesondere das Kulturmedium oder der Reifungscocktail IL-1β, IL-6, PGE₂ und TNFα enthält oder der Reifungscocktail Monocyten-konditioniertes Medium (MCM) oder MCM mit zusätzlichem PGE₂ ist.

5. Verfahren nach Anspruch 4, wobei die unreifen DC in Gegenwart von 0,1 bis 100 ng/ml IL-1β, 0,1 bis 100 ng/ml IL-6, 0,1 bis 10 µg/ml PGE₂ und 0,1 bis 100 ng/ml TNF-α kultiviert werden.

6. Verfahren nach Anspruch 4 oder 5, wobei die Wirkstoffe IL-1β, IL-6, PGE₂ und TNFα, die den DC zur Ausreifung zugesetzt werden, aus gereinigten Zubereitungen der einzelnen Wirkstoffe stammen.

7. Verfahren nach einem oder mehreren der Ansprüche 1 bis 6, wobei
(i) die Reifung für wenigstens 1 Stunde, vorzugsweise wenigstens 6 Stunden, erfolgt und/oder
(ii) während der Reifung der Reifungscocktail des Kulturmediums ausgetauscht wird und/oder zusätzliche Reifungsstimuli dem Kulturmedium zugegeben werden; und/oder
(iii) dem Kulturmedium Immun-/Reifungsmodulatoren, insbesondere ausgewählt aus IL-10, Fumarsäure und Ester davon, Mycophenolatmofetil und Vitamin D3, zugegeben werden.

8. Verfahren nach einem der vorhergehenden Ansprüche, wobei die Polyolverbindungen ausgewählt sind aus Glukose, Dextran, Sucrose, Ethylenglykol, Erythrit, D-Ribit, D-Mannit, D-Sorbit, Inosit und D-Laktose, wobei Glukose besonders bevorzugt ist.

9. Verfahren nach Anspruch 8, wobei das Einfriermedium 5-10% (w/v) Glukose enthält.

10. Verfahren nach einem der vorhergehenden Ansprüche, wobei die Zellen in einer Konzentration von 1 x 10⁶ bis 100 x 10⁶ Zellen/ml eingefroren werden.

11. Verfahren nach Anspruch 10, wobei die Zellen in einer Konzentration von 5 x 10⁶ bis 20 x 10⁶ Zellen/ml eingefroren werden.

12. Verfahren nach einem oder mehreren der Ansprüche 2 bis 11, wobei die DC vor dem Einfrieren mit Antigen oder Antigen-Antikörperkomplexen beladen werden.

13. Verfahren nach einem oder mehreren der Ansprüche 2 bis 12, wobei das Antigen, mit dem die DC beladen werden, ein Protein oder ein Fragment davon mit wenigstens 8 Aminosäuren ist und insbesondere das Protein oder Fragment in einer Konzentration von 0,01 bis 1000 µM zum Kulturmedium, gegeben wird, vorzugsweise während IL-1β, IL-6, PGE₂ und TNFα im Kulturmedium enthalten sind.

14. Verfahren nach einem der Ansprüche 1, 2 oder 12, wobei die DC mit einem DNA- oder RNA-Molekül beladen werden, das für das Antigen kodiert.

15. Verfahren nach einem oder mehreren der Ansprüche 2 bis 12, wobei in dem Antigen-Antikörperkomplex als Antigen ein Proteinfragment, Zellen oder Zellbruchstücke oder Nukleinsäuresequenzen dienen und der Antikörper aus der Klasse IgG oder IgE ist.

16. Verfahren nach einem oder mehreren der Ansprüche 1 bis 15, wobei die DC vor dem Einfrieren oder nach dem Auftauen mit einem Molekül in Kontakt gebracht werden, das die Apoptose hemmen kann, wobei das Molekül, das die Apoptose hemmen kann, ausgewählt ist aus CD40-Ligand, TRANCE und RANKL.

17. Verfahren nach einem oder mehreren der Ansprüche 1 bis 16, wobei nach Einfrieren und Auftauen der DC mehr als 75%, bevorzugt mehr als 85% überlebende DC, bezogen auf die Anzahl eingefrorener DC erhalten werden.

## Claims

1. A method for the preparation of cryoconserved mature dendritic cells that are immediately usable after thawing, comprising
a) providing immature dendritic cells (DC);
b) culturing the immature DCs in a culture medium containing a maturing cocktail with one or more maturing stimulants to obtain mature DCs;
c) freezing the mature DCs in a freezing medium which contains autologous serum or autologous plasma, from 5 to 25% (v/v) of DMSO and from 2 to 30% (w/v) of one or more polyol compounds.

2. The method according to claim 1, wherein said DCs are loaded with an antigen or antigen-antibody complex prior to said freezing or after rethawing the frozen DCs.

3. The method according to claim 1 or 2, wherein
(i) said immature DCs are prepared from CD14+ mononuclear cells (monocytes) or from CD34+ cells; or
(ii) said immature DCs are isolated directly from blood; or
(iii) said immature DCs or their precursor cells are obtained from leucapheresates; or
(iv) said immature DCs or their precursor cells are obtained from fresh blood or bone marrow.

4. The method according to one or more of claims 1 to 3, wherein said maturing stimulants are selected from IL-1, such as IL-1β, IL-6, TNF-α, prostaglandins, such as PGE₂, IFN-α, lipopolysaccharides and other bacterial cell products, such as MPL (monophosphoryllipid A) and lipoteichoic acid, phosphorylcholine, calcium ionophores, phorbol esters, such as PMA, heat-shock proteins, nucleotides, such as ATP, lipopeptides, artificial ligands for Toll-like receptors, double-stranded RNA, such as poly-I:C, immunostimulatory DNA sequences and CD40 ligand, said culture medium or said maturing cocktail contain, in particular, IL-1β, IL-6, PGE₂ and TNFα, or said maturing cocktail is monocyte-conditioned medium (MCM) or MCM supplemented with PGE₂.

5. The method according to claim 4, wherein said immature DCs are cultured in the presence of from 0.1 to 100 ng/ml IL-1β, from 0.1 to 100 ng/ml IL-6, from 0.1 to 10 µg/ml PGE₂, and from 0.1 to 100 ng/ml TNF-α.

6. The method according to claim 4 or 5, wherein the active substances IL-1β, IL-6, PGE₂ and TNFα, which are added to the DCs for maturing, are derived from purified formulations of the individual active substances.

7. The method according to one or more of claims 1 to 6, wherein
(i) the maturing is performed for at least one hour, preferably at least 6 hours; and/or
(ii) during the maturing, the maturing cocktail of the culture medium is replaced, and/or additional maturing stimulants are added to the culture medium; and/or
(iii) immune/maturing modulators, especially those selected from IL-10, fumaric acid and esters thereof, mycophenolate mofetil and vitamin D3, are added to the culture medium.

8. The method according to any of the preceding claims, wherein said polyol compounds are selected from glucose, dextran, sucrose, ethylene glycol, erythritol, D-ribitol, D-mannitol, D-sorbitol, inositol and D-lactose, wherein glucose is particularly preferred.

9. The method according to .claim 8, wherein said freezing medium contains from 5 to 10% (w/v) of glucose.

10. The method according to any of the preceding claims, wherein said cells are frozen in a concentration of from 1 x 10⁶ to 100 x 10⁶ cells/ml.

11. The method according to claim 10, wherein said cells are frozen in a concentration of from 5 x 10⁶ to 20 x 10⁶ cells/ml.

12. The method according to one or more of claims 2 to 11, wherein said DCs are loaded with an antigen or with antigen-antibody complexes prior to being frozen.

13. The method according to one or more of claims 2 to 12, wherein said antigen with which the DCs are loaded is a protein or a fragment thereof having at least 8 amino acids, in particular said protein or fragment being added to the culture medium, in a concentration of from 0.01 to 1000 µM, preferably while It-1β, IL-6, PGE₂ and TNFα are contained in the culture medium.

14. The method according to one of claims 1, 2 or 12, wherein said DCs are loaded with a DNA or RNA molecule which codes for said antigen.

15. The method according to one or more of claims 2 to 12, wherein a protein fragment, cells or cell fragments or nucleic acid sequences serve as the antigen in said antigen-antibody complex, and the antibody is one of class IgG or IgE.

16. The method according to one or more of claims 1 to 15, wherein said DCs, prior to being frozen or after being rethawed, are contacted with a molecule which is capable of inhibiting apoptosis, wherein said molecule capable of inhibiting apoptosis is selected from CD40 ligand, TRANCE and RANKL.

17. The method according to one or more of claims 1 to 16, wherein more than 75%, preferably more than 85%, of surviving DCs, based on the number of frozen DCs, are obtained after said freezing and rethawing of the DCs.

## Revendications

1. Procédé de fabrication de cellules dendritiques matures, cryoconservées, immédiatement disponibles après décongélation, comprenant
a) la mise à disposition de cellules dendritiques (CD) immatures ;
b) la culture des CD immatures dans un milieu de culture comprenant un cocktail inducteur de maturation avec un ou plusieurs agent(s) stimulant la maturation, et l'obtention de CD matures ;
c) la congélation des CD matures dans un milieu de congélation comprenant du sérum autologue ou du plasma autologue, 5 à 25 % (en volume) de DMSO et 2 à 30 % (en poids/volume) d'un ou plusieurs composé(s) de type polyol.

2. Procédé selon la revendication 1, dans lequel les CD sont chargées en antigène ou en complexe antigène-anticorps avant la congélation ou après redécongélation des CD congelées.

3. Procédé selon la revendication 1 ou 2, dans lequel
i) les CD immatures sont fabriquées à partir de cellules CD 14 positives, de cellules mononucléaires (monocytes) ou à partir de cellules CD34 positives ; ou
ii) les CD immatures sont directement isolées à partir de sang ; ou
iii) les CD immatures ou leurs cellules précurseurs sont obtenues à partir du produit d'une leucaphérèse ; ou
(iv) les CD immatures ou leurs cellules précurseurs sont obtenues à partir de sang frais ou de moelle osseuse.

4. Procédé selon une ou plusieurs des revendications 1 à 3, dans lequel les agents stimulant la maturation sont choisis parmi l'IL-1, telle que l'IL-1β, l'IL-6, le TNF-α, la prostaglandine telle que la PGE₂, l'IFN-α, des lipopolysaccharides et d'autres produits cellulaires bactériens tels que le MPL (le lipide monophosphoryle A) et l'acide lipotéichoïque, la phosphorylcholine, l'ionophore calcique, l'ester de phorbol tel que le PMA, des protéines de choc thermique, des nucléotides tels que l'ATP, des lipopeptides, des ligands artificiels pour les récepteurs de type Toll, l'ARN double brin tel que le poly-I:C, des séquences d'ADN immunostimulantes et le ligand CD40 et, en particulier, le milieu de culture ou le cocktail inducteur de maturation contient l'IL-1β, l'IL-6, la PGE₂ et le TNF-α, ou le cocktail inducteur de maturation est un milieu conditionné de monocytes (MCM) ou un milieu MCM additionné de PGE₂.

5. Procédé selon la revendication 4, dans lequel les CD immatures sont cultivées en présence de 0,1 à 100 ng/ml d'IL-1β, de 0,1 à 100 ng/ml d'IL-6, de 0,1 à 10 µg/ml de PGE₂ et de 0,1 à 100 ng/ml de TNF-α.

6. Procédé selon la revendication 4 ou 5, dans lequel les principes actifs IL-1β, IL-6, PGE₂ et TNF-α qui sont ajoutés aux CD pour la maturation proviennent de préparations purifiées des principes actifs individuels.

7. Procédé selon une ou plusieurs des revendications 1 à 6, dans lequel
i) la maturation s'effectue pendant au moins 1 heure, de préférence au moins 6 heures, et/ou
ii) le cocktail inducteur de maturation du milieu de culture est remplacé au cours de la maturation et/ou des agents stimulant la maturation supplémentaires sont ajoutés au milieu de culture ; et/ou
iii) des immunomodulateurs et des modulateurs de maturation, choisis en particulier parmi l'IL-10, l'acide fumarique et ses esters, le mycophénolate mofétil et la vitamine D3 sont ajoutés au milieu de culture.

8. Procédé selon l'une des revendications précédentes, dans lequel les composés de type polyol sont choisis parmi le glucose, le dextrane, le saccharose, l'éthylène glycol, l'érythritol, le D-ribitol, le D-mannitol, le D-sorbitol, l'inositol et le D-lactose, le glucose étant particulièrement préféré.

9. Procédé selon la revendication 8, dans lequel le milieu de congélation comprend 5 à 10 % (poids/volume) de glucose.

10. Procédé selon l'une quelconque des revendications précédentes, dans lequel les cellules sont congelées à raison d'une concentration de 1 x 10⁶ à 100 x 10⁶ cellules/ml.

11. Procédé selon la revendication 10, dans lequel les cellules sont congelées à raison d'une concentration de 5 x 10⁶ à 20 x 10⁶ cellules/ml.

12. Procédé selon une ou plusieurs des revendications 2 à 11, dans lequel les CD sont chargées en antigène ou en complexes antigène-anticorps avant la congélation.

13. Procédé selon une ou plusieurs des revendications 2 à 12, dans lequel l'antigène avec lequel les CD sont chargées est une protéine ou un fragment de protéine comportant au moins 8 acides aminés et, en particulier, la protéine ou le fragment est incorporé au milieu de culture à raison d'une concentration de 0,01 à 1 000 µM, de préférence alors que l'IL-1β, l'IL-6, la PGE₂ et le TNF-α sont présents dans le milieu de culture.

14. Procédé selon l'une des revendications 1, 2 ou 12, dans lequel les CD sont chargées avec une molécule d'ADN ou d'ARN codant pour l'antigène.

15. Procédé selon une ou plusieurs des revendications 2 à 12, dans lequel on utilise en tant qu'antigène dans le complexe antigène-anticorps un fragment de protéine, des cellules ou des fragments de cellules ou encore des séquences d'acides nucléiques, et dans lequel l'anticorps appartient à la classe des IgG ou des IgE.

16. Procédé selon une ou plusieurs des revendications 1 à 15, dans lequel les CD sont mises en contact, avant la congélation ou après décongélation, avec une molécule capable d'empêcher l'apoptose, la molécule capable d'empêcher l'apoptose étant choisie parmi les ligands CD-40, TRANCE et RANKL.

17. Procédé selon une ou plusieurs des revendications 1 à 16, dans lequel on obtient, après congélation et décongélation des CD, plus de 75 %, de préférence plus de 85 %, de CD survivantes, par rapport au nombre de CD congelées.
